(19) Europäisches Patentamt
European Patent Office
Office européen des brevets

(11) EP 2 243 470 A1

(12) # EUROPÄISCHE PATENTANMELDUNG

(43) Veröffentlichungstag:
**27.10.2010 Patentblatt 2010/43**

(51) Int Cl.:
***A61K 9/16*** *(2006.01)* ***A61K 9/19*** *(2006.01)*
***A61K 9/20*** *(2006.01)* ***A61K 9/28*** *(2006.01)*

(21) Anmeldenummer: **10160418.9**

(22) Anmeldetag: **20.04.2010**

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**
Benannte Erstreckungsstaaten:
**AL BA ME RS**

(30) Priorität: **22.04.2009 EP 09158477**

(71) Anmelder: **Dr. Suwelack Skin & Health Care AG**
**48727 Billerbeck (DE)**

(72) Erfinder:
- **Rauert, Daniel**
**48720 Rosendahl Darfeld (DE)**
- **Elsinghorst, Claudia**
**48727 Billerbeck (DE)**
- **Niehues, Anke**
**48607 Ochtrup (DE)**
- **Malessa, Ralf**
**45134 Essen (DE)**
- **Frahling, Stefan**
**48727 Billerbeck (DE)**

(74) Vertreter: **Gille Hrabal Struck Neidlein Prop Roos**
**Brucknerstraße 20**
**40593 Düsseldorf (DE)**

(54) **Gefriergetrockneter beschichteter Formkörper**

(57) Die Erfindung betrifft gefriergetrocknete Formkörper enthaltend einen oder mehrere Wirkstoffe und gegebenenfalls einen oder mehrere Gerüstbildner, gegebenenfalls einen oder mehrere Hilfsstoffe, sowie eine Beschichtung, enthaltend mindestens einen Filmbildner. Weiterhin betrifft die Erfindung Verfahren zur Herstellung dieser gefriergetrockneten Formkörper, die Kombination solcher gefriergetrockneten Formkörper in Kit-of-parts Anordnungen zusammen mit wässrigen Lösungen sowie die Verwendung der gefriergetrockneten Formkörper und der Kit-of-parts Kombinationen zur pharmazeutischen und kosmetischen Anwendung.

EP 2 243 470 A1

## Beschreibung

Die Erfindung betrifft gefriergetrocknete Formkörper enthaltend einen oder mehrere Wirkstoffe und gegebenenfalls einen oder mehrere Gerüstbildner, gegebenenfalls einen oder mehrere Hilfsstoffe, sowie eine Beschichtung, enthaltend mindestens einen Filmbildner. Weiterhin betrifft die Erfindung Verfahren zur Herstellung dieser gefriergetrockneten Formkörper, die Kombination solcher gefriergetrockneten Formkörper in Kit-of-parts Anordnungen zusammen mit wässrigen Lösungen sowie die Verwendung der gefriergetrockneten Formkörper und der Kit-of-parts Kombinationen zur pharmazeutischen und kosmetischen Anwendung.

Mit der Gefriertrocknung steht eine Methode zur Verfügung, die es ermöglicht, schnell hydratisierende Materialien herzustellen. Im Gefriertrocknungsprozess wird dabei, in Abhängigkeit des Wassergehaltes der zu trocknenden Substanz, eine hoch poröse Matrix erzeugt. Dabei bilden sich in der zu trocknenden Matrix-Lösung aus dem enthaltenen Wasser während des Gefrierprozesses Eiskristalle, die nach der Sublimation im Gefriertrocknungsprozess die Porenstruktur des Materials bestimmen. Diese Porenstruktur resultiert in einer großen inneren Oberfläche, welche für das Rehydratationsverhalten des gefriergetrockneten Materials maßgeblich ist. Dies führt in Abhängigkeit der Materialzusammensetzung in der Regel zu sehr kurzen Rehydratationszeiten. Dabei ist die Materialzusammensetzung außerdem maßgeblich für die mechanische Stabilität der gefriergetrockneten porösen Matrices. Insbesondere bei der Herstellung von gefriergetrockneten wirkstoffhaltigen Materialien oder Formkörpern, wie sie für die pharmazeutische oder kosmetische Verwendung bekannt und verbreitet sind, spielt dabei die mechanische Stabilität und damit Handhabbarkeit der Materialien nach dem Gefriertrocknungsprozess eine zentrale Rolle. Um formstabile Wirkstoff-Matrices ausbilden zu können, müssen der zu trocknenden Zusammensetzung üblicherweise neben den eigentlichen Wirkstoffen auch Hilfsstoffe hinzugefügt werden, welche dem gefriergetrockneten Endprodukt eine gewisse mechanische Festigkeit verleihen. Hierzu werden meist für kosmetische oder pharmazeutische Anwendungsgebiete unbedenkliche gerüstbildende Substanzen oder Polymere, sogenannte Gerüstbildner, insbesondere natürliche Polymere z. B. aus der Gruppe der Hydrokolloide wie insbesondere solche auf der Basis von Kollagen oder auf der Basis pflanzlicher oder tierischer Polysaccharide eingesetzt. Allgemein gilt dabei, je höher der Anteil an gerüstbildenden Polymeren, umso höher die mechanische Festigkeit des gefriergetrockneten Produktes und umso kleiner die Hydratationsgeschwindigkeit. Hohe Polymerkonzentrationen führen also einerseits zu mechanisch stabilen und belastbaren, abriebfesten und somit staubfreien Produkten mit guter Handhabbarkeit, welche aber andererseits bedingt durch das Quellverhalten der darin enthaltenen Polymere länger zur vollständigen Benetzung benötigen. Niedrige Gerüstbildnerkonzentrationen hingegen führen zu Produkten, die sich innerhalb kürzester Zeit vollständig benetzen lassen, jedoch nicht über ausreichende mechanische Stabilität und Festigkeit verfügen und somit äußerst fragile und staubende Produkte liefern.

Somit stellt das erhältliche gefriergetrocknete Produkt bis dato immer einen Kompromiss zwischen schneller Auflösung und hinreichender mechanischer Stabilität dar.

Um gefriergetrocknete Materialien mit geringer mechanischer Festigkeit zu stabilisieren, bietet sich an, eine durchgehende, stabile Schutzschicht auf dem Material aufzubringen. Dadurch können Formkörper mit einer Rezepturverteilung, die aus einem inneren wirkstoffhaltigen Kern mit sehr geringem oder vollständig ohne Strukturpolymeranteil und einer äußeren Polymerschicht zur Stabilisierung des instabilen wirkstoffhaltigen Kerns erhalten werden.

Dabei ist das Überziehen von Formkörpern mit filmbildenden bzw. strukturgebenden Polymeren zur Veränderung der physikalisch/chemischen Eigenschaften dieser Formkörper, insbesondere auch zur Stabilisierung und Beeinflussung des Auflösungsverhaltens solcher Formkörper aus dem Stand der Technik grundsätzlich bekannt und im pharmazeutischen Bereich z. B. in der Technologie der Tablettenüberzüge verbreitet und in einem weiten Anwendungsspektrum genutzt. So können Tabletten bekanntermaßen mit säurefesten Überzügen hergestellt werden, um magensaftresistente Wirkstoffformulierungen zu erhalten. Auch Überzüge, die eine kontrollierte bzw. verzögerte Wirkstofffreisetzung ermöglichen, zur Herstellung sogenannter Sustained-release Formulierungen sind bekannt. Desweiteren werden bei der Tablettenherstellung Überzüge generell zur Verhinderung von Staubentwicklung, Feuchtigkeitsaufnahme und anderen instabilisierenden Einflüssen sowie generell zur mechanischen Stabilisierung der Materialien verwendet.

Dabei erfolgt die Herstellung üblicherweise in einem dreistufigen Prozess. Nach der Herstellung der Tabletten werden diese in einem zweiten Schritt mit einem in einem Lösungsmittel gelösten Überzug versehen und in einem dritten Schritt wird das Lösungsmittel z. B. durch Trocknung oder Verdampfen entfernt. Um diesen Prozess zum Beschichten oder Überziehen anwenden zu können, ist eine hinreichende Stabilität des zu überziehenden Materials Voraussetzung. Dabei muss das Material zum einen herstellungsbedingt gegen mechanische Beanspruchung stabil sein, da das Überziehen in der Regel in rotierenden Trommeln oder ähnlichen beweglichen Einrichtungen erfolgt, um einen gleichmäßigen, homogenen Überzug zu erhalten. Zum anderen muss das Material stabil sein gegenüber dem aufgebrachten Überzugsmittel. Dies ist insbesondere relevant für schnell-lösliche Materialien, die mit einem Überzug versehen werden sollen. Hier liegt die besondere Herausforderung darin, dass das Material, das sich durch eine schnelle Löslichkeit auszeichnen soll, gegenüber dem Überzugsmaterial so stabilisiert werden kann, dass hier die schnelle Löslichkeit gegenüber den Überzugslösungsmitteln nicht zum Tragen kommt oder durch geeignete Methoden soweit verzögert werden kann, dass das Material bis nach Abschluss der Beschichtung formstabil bleibt.

Aus dem Stand der Technik sind verschiedene leicht-lösliche gefriergetrocknete wirkstoffhaltige Formkörper und deren Herstellungsverfahren bekannt. So beschreiben die JP 2004-149468, die EP 0081912 oder auch die US 4,305,502 feste durch gerüstbildende Polymere stabilisierte Wirkstoffzusammensetzungen, die sich durch eine schnelle Löslichkeit auszeichnen. Dabei werden diese Zusammensetzungen jedoch direkt in den Formen bzw. in der Endverpackung (z. B. Flaschen oder Blister) getrocknet und verpackt, was mit der geringen mechanischen Stabilität der durch diese Verfahren erhältlichen Formkörper zusammenhängt. Lose erhältliche Einzelformkörper und somit entsprechend mechanisch stabile Formkörper sind hiernach nicht erhältlich. Da die Materialien direkt in der Endverpackung verbleiben, ist außerdem offensichtlich keine gleichmäßige Überziehung des Materials vorgesehen und aufgrund der geringen Stabilität auch nicht möglich.

Feste, schnell-lösliche Wirkstoffzubereitungen, die mit gerüstbildenden Polymeren stabilisiert sind, sind darüber hinaus bekannt aus der DE 69227467 oder der JP 2003-238693, sowie aus der WO 04/011537 und der WO 05/073296, wobei die hier beschriebenen Zubereitungen einen ausreichend hohen stabilisierenden Polymeranteil in der Zusammensetzung aufweisen, so dass eine zusätzliche stabilisierende Beschichtung offensichtlich nicht notwendig und auch nicht beschrieben ist. Durch den vergleichsweise hohen Anteil an Gerüstbildnern in den hier offenbarten Zusammensetzungen ergibt sich jedoch unmittelbar der oben beschriebene Nachteil der vergleichsweise schlechteren Löslichkeit. So lösen sich die Zubereitungen nach JP 2003-238693 lediglich durch mechanische Einwirkung z. B. durch Verreiben mit dem Finger, die Zusammensetzungen der WO 04/011537 und der WO 05/073296 verweisen lediglich auf eine gute Löslichkeit oder Dispergierbarkeit ohne gesonderte Aussagen über die Auflösungsgeschwindigkeit zu treffen. Die in der DE 69227467 beschriebenen Zubereitungen hingegen verfügen offensichtlich über außerordentlich gute Auflösungsgoschwindigkoiten, dabei erhalten diese ihre hohe mechanische Stabilität allerdings über den hohen Anteil an Füllstoffen wie Lactose oder Mannitol (mehr als 50 Gew.-%), wodurch Zubereitungen mit besonders hohem Wirkstoffanteil nicht erhältlich sein können. Eine Stabilisierung solcher leicht-löslicher Zubereitungen mit hohem Wirkstoffanteil und geringem Anteil strukturgebender Hilfsstoffe durch Beschichtung ist somit nicht Gegenstand dieser Offenbarungen.

Feste Wirkstoff-Zubereitungen, die mit polymeren Strukturbildnern stabilisiert sind und sich bei Flüssigkeitszufuhr auflösen und die darüber hinaus durch eine Beschichtung oder ein Coating in ihren physikalisch/chemischen Eigenschaften modifiziert sind, sind in der US 5,843,347 oder auch in der US 5,578,307, US 5,405,616, EP 0701815 und DE 4201179 beschrieben. Dabei sind in diesen Dokumenten jedoch Überzüge offenbart, die die Wirkstoffzubereitungen soweit in ihrem Auflösungsverhalten beeinflussen, dass keine schnelle Löslichkeit mehr erhalten werden kann. Die Überzüge sind vielmehr zur Herstellung von magensaftresistenten Zubereitungen oder zur Bereitstellung von Sustained-release Zubereitungen vorgesehen, was zwangsläufig eine deutlich verringerte Löslichkeit z. B. in wässrigen oder physiologischen Medien wie z. B. Speichel bei der oralen Applikation mit sich führt. Die hier offenbarten Zubereitungen geben somit keine Hinweise auf eine geeignete Überzugstechnik, die zwar einerseits zu einer Stabilisierung der festen wirkstoffhaltigen Formkörper oder Zubereitungen führt und andererseits dennoch eine schnelle Löslichkeit gewährleistet bzw. erhält. Darüber hinaus enthalten alle hier beschriebenen Zubereitungen zwingend Gerüstbildner, die aus der Gruppe der Proteine ausgewählt sind. Derartige proteinogene Gerüstbildner sind dabei maßgeblich für die Stabilität der gefriergetrockneten Zusammensetzungen, da diese im Gefriertrocknungsprozess bekanntermaßen eine Vernetzung, die sogenannte Dehydrothermalvernetzung, erfahren. Durch diese Vernetzung wird ein Material erhalten, welches ausreichend stabil ist, um nach dem Gefriertrocknungsprozess, also im getrockneten Zustand, mit dem Filmüberzug versehen zu werden. Eine Beschichtung im gefrorenen Zustand wird nicht beschrieben. Durch die für dieses Verfahren notwendige mechanische Stabilität der gefriergetrockneten Pellets ist außerdem ein ausreichend hoher Anteil dieser gerüstbildenden Polymere notwendig und Zusammensetzungen mit einen Wirkstoffgehalt < 50 Gew.% können durch das hier beschriebene Verfahren nicht erhalten werden. Mit Erhöhung des Wirkstoffgehaltes geht ein Verlust der mechanischen Stabilität des gefiergetrockneten Formkörpers einher, der es unmöglich macht, insbesondere bei Verwendung von hydrophilen Wirkstoffen, diese nachfolgend zu beschichten, da diese beim Beschichtungsversuch sofort kollabieren.

Die GB 1206033 beschreibt außerdem gefriergetrocknete Nahrungsmittel-Formkörper, insbesondere gefriergetrocknete Eiscreme, die beispielsweise mit Schokolade, die prinzipiell als Filmbildner bezeichnet werden könnte, überzogen werden kann. Allerdings erfolgt auch hier die Beschichtung erst nach dem Schritt der Gefriertrocknung, die in Form-Tabletts durchgeführt werden kann, die ggf. mit einem Filmbildner-Coating versehenen wurden. Ein mit einem Filmbildner beschichteter wirkstoffhaltiger Formkörper wird hier jedoch nicht offenbart.

Somit sind die vorstehend beschriebenen Methoden zum Coating von leicht-löslichen, festen Wirkstoff-Zusammensetzungen lediglich für solche Zubereitungen geeignet, die an sich bereits über eine hinreichende mechanische Stabilität verfügen. Will man jedoch besonders fragile und an sich mechanisch instabile Wirkstoffzusammensetzungen, insbesondere solche, die über keinen oder nur einen extrem geringen Anteil an strukturgebenden oder gerüstbildenden polymeren Hilfsstoffen bzw. über einen besonders hohen Wirkstoffgehalt verfügen, mit einem Coating stabilisieren, um eine erhöhte mechanische Festigkeit zu erreichen, so ergibt sich zum einen das Problem, dass die an sich mechanisch instabilen festen Zubereitungen den meisten herkömmlichen Coatingverfahren aufgrund der dabei entstehenden mechanischen Belastung nicht zugänglich sind. Zum anderen dürfen solche schnell-löslichen festen Zubereitungen aufgrund

ihrer hohen Empfindlichkeit gegenüber lösenden Medien nicht mit dem Überzugsmittel derart reagieren, dass die Auflösung bereits beim Stabilisierungs- bzw. Überzugsverfahren eintritt. Weiterhin müssen die verwendeten Überzugsmaterialien so beschaffen sein, dass auch nach dem Coating eine schnelle Löslichkeit des beschichteten Formkörpers erhalten bleibt. Dies ist insbesondere dann problematisch, wenn der fertige gefriergetrocknete Formkörper abschließend mit einem Coating versehen werden soll, wie es beispielsweise in den oben genannten Verfahren beschrieben ist. Dabei erfolgt in allen vorstehend beschriebenen Verfahren die Beschichtung an der festen, getrockneten Zubereitungsform.

Versucht man nun, gefriergetrocknete Formkörper mit den gewünschten Eigenschaften hoher Wirkstoffanteil, wenig gerüstbildende Hilfsstoffe, hohe Auflösungsgeschwindigkeit und hohe bzw. hinreichende mechanische Stabilität zur Bereitstellung von losen Einzelformkörpern, insbesondere zur Verwendung als single-unit Dosisform durch ein Coating zu stabilisieren, so stellt sich heraus, dass hydrophile Überzugslösungen wie z. B. auf der Basis von Glycerin zum einen die Hygroskopizität, sprich die Feuchtigkeitsempfindlichkeit des Endproduktes erhöhen und darüber hinaus zu einem instabilen Coating führen, was sich insbesondere in Schmier- und Abriebfilmen auf Verpackungsmaterialien äußert. Überzugslösungen auf wässriger Basis sind für solche gefriergetrockneten Zubereitungen aus den genannten Gründen der hohen Auflösungsgeschwindigkeit und damit extrem hohen Empfindlichkeit gegenüber wässrigen Lösungsmitteln ungeeignet. Verwendet man hydrophobe Coatings z. B. auf der Basis von Fetten und Ölen, wie z. B. Neutralöl oder auf Basis von hydrophoben Polymeren wie z. B. in der JP 54105289 beschrieben, so wird dadurch das Auflösungsverhalten der wirkstoffhaltigen Formkörper insbesondere in wässrigen Medien oder physiologischen Flüssigkeiten wie z. B. Speichel so weit herabgesetzt, dass die Auflösungsgeschwindigkeit sehr stark verzögert wird. Dies ist insbesondere nachteilig, wenn eine schnelle Wirkstofffreisetzung und somit eine schnelle Verfügbarmachung der Wirkstoffe gewünscht ist. Aus diesen Gründen ist ein Coating auf Basis einer hydrophilen, wässrigen Coatingzusammensetzung bevorzugt. Eine solche ist jedoch aus den genannten Gründen für die vorliegend gewünschten Formkörper bisher nicht beschrieben und aus den genannten Gründen bisher nicht erhältlich.

Eine Möglichkeit, ein hydrophiles Coating auf einem schnell-löslichen Formkörper zu erhalten, besteht darin, das Coating vor der Gefriertrocknung auf den gefrorenen Formkörper aufzubringen. Der Vorteil liegt darin, dass die Eisstruktur der Zusammensetzung die ausreichende mechanische Stabilität verleiht, damit der Formkörper der mechanischen Beanspruchung während des Coating-Verfahrens standhält. Da sich die besonders schnelle Löslichkeit der Zusammensetzungen maßgeblich aus der Porenstruktur der gefriergetrockneten Zusammensetzungen ergibt, und somit dieses schnelle Löslichkeitspotential im gefrorenen Zustand noch nicht in dem Maße vorliegt, kann durch Coating der gefrorenen Zusammensetzungen zum anderen auch das Problem der vorzeitigen Auflösung oder Feuchtigkeitsinstabilität der gefriergetrockneten Formkörper mit dem Coating-Lösemittel umgangen werden. Das Coating-Lösemittel wird dabei zusammen mit dem Lösemittel der Zusammensetzung, üblicherweise dem Wassergehalt der Wirkstoffzusammensetzung, im anschließenden Schritt der Gefriertrocknung entfernt. Dies erhöht außerdem die Effizienz des Verfahrens, da gegenüber Verfahren, in denen das Endprodukt einem Coatingschritt unterworfen wird, der zusätzliche Arbeitsschritt zum Entfernen des Coating-Lösemittels eingespart wird. Darüber hinaus ist durch die Methode der Beschichtung der gefrorenen Formkörper sichergestellt, dass feuchtigkeitsempfindliche Wirkstoffe durch die tiefen Temperaturen im gefrorenen Formkörper geschützt sind, wohingegen solche empfindlichen Wirkstoffe in bereits getrockneten Zusammensetzungen bei Kontakt mit dem Coating-Lösemittel diesem eine gewisse Zeit ausgesetzt sind, was gegebenenfalls zu deutlichen Aktivitätsverlusten bei feuchtigkeitszersetzlichen Wirkstoffen führen kann.

Weiterhin ergibt sich durch das Verfahren der Beschichtung von gefrorenen Formkörpern und anschließender Gefriertrocknung eine hohe Variabilität in der Wahl der Rezeptur der Wirkstoffzusammensetzung. Die gerüstbildenden Strukturpolymere können in deutlich geringeren Konzentrationen eingesetzt werden. Idealerweise kann sogar ganz auf deren Zusatz verzichtet werden, so dass trockene Formkörper erhältlich sind, die ohne Strukturpolymere im inneren Bereich der Formkörper auskommen und die lediglich durch eine dünne polymere, filmbildende Überzugsschicht mechanisch stabilisiert sind.

Schnell-lösliche feste Zubereitungen, die im gefrorenen Zustand gecoatet werden, werden in der bereits zitierten JP 54105289 erwähnt, worin allerdings Eispellets gecoatet werden, ohne dass diese einem anschließenden Gefriertrocknungsschritt unterzogen werden. Darüber hinaus wird ein hydrophobes Coating verwendet, was aus besagten Gründen für die Applikation von Wirkstoffen in wässrigen Systemen nachteilig ist. Darüber sind hydrophobe Coatings für gefrorene Formkörper, die anschließend einer Gefriertrocknung unterzogen werden insofern ungeeignet, als durch das hydrophobe Coating der Flüssigkeitsaustritt aus dem inneren der Zusammensetzung bei der Sublimation verhindert bzw. stark eingeschränkt wird, was sich nachteilig auf den Gefriertrocknungsprozess auswirkt.

In der DE 10248314 bzw. der korrespondierenden WO 2004/035023 werden schnell-lösliche gefriergetrocknete Formkörper aus Wirkstoff-Gerüstbildner-Mischungen für die äußere Anwendung beschrieben. Die Möglichkeit, diese Formkörper im gefrorenen Zustand vor der Gefriertrocknung einer Oberflächenbeschichtung zu unterziehen wird erwähnt. Es werden jedoch lediglich solche Überzüge zum Aufbringen einer Wirkstoff- oder Farbschicht erwähnt und solche, die durch Vernetzung der im Formkörper enthaltenen Strukturpolymere auf Basis von Alginat die Auflösungsgeschwindigkeit der Formkörper verringern. Dabei wird die Möglichkeit der mechanischen Stabilisierung der Formkörper durch die Beschichtung, insbesondere auch durch eine Beschichtung mit einem Filmbildner, nicht offenbart, zumal eine

solche aufgrund der in den dort offenbarten Wirkstoffzusammensetzungen enthaltenen hohen Anteile an gerüstbildenden Strukturpolymeren von mindestens 10 Gew.-%, bevorzugt 15 - 100 Gew.-% bereits durch die Gerüstbildner in ausreichendem Maß gegeben ist. Die Möglichkeit, stabile gefriergetrocknete Formkörper mit hohen Wirkstoffgehalten und extrem geringen Gerüstbildneranteilen bei ausreichender mechanischer Stabilität durch ein dünnes Filmbildner-Coating zu erhalten, wird in der DE 10248314 oder der korrespondierenden WO 2004/035023 nicht offenbart. Insbesondere in Anbetracht der bisher begrenzten Möglichkeiten bei der Einarbeitung einer Reihe von wichtigen und hochpotenten Wirkstoffen für die kosmetische oder pharmazeutische Anwendung, die dafür bekannt sind, äußerst instabil zu sein gegenüber äußeren Einflüssen wie Licht, Temperatur, Oxidation oder Feuchtigkeit, in kosmetische und pharmazeutische Zubereitungen, besteht ein weiteres zentrales Interesse daran, solche hochpotenten und hochgradig instabilen und abbaugefährdeten Wirkstoffe in hoher Konzentration in eine Form zu bringen, die eine hohe und langfristige Stabilität und damit einhergehend eine gute Lagerfähigkeit, optimale und reproduzierbare Wirkstoffgehalt-Bereitstellung über die gesamte Lager und Verabreichungszeit und somit höchstmögliche Sicherheit und Dosierbarkeit bei der Verwendung ermöglichen.

Dabei ist neben einer wirksamen Stabilisierung der Wirkstoffe aber auch die Bereitstellung dieser in einer optimal geeigneten und auf den jeweiligen Anwendungszweck bestmöglich abgestimmten Darreichungsform von besonderem Interesse. Die Wahl der geeigneten Darreichungsform hängt dabei insbesondere ab von der Art und dem Ort der Applikation, der Zielgruppe und deren Eigenheiten, der Art und Höhe der Dosierung der Wirkstoffe oder ihrer Darreichungsform sowie beispielsweise auch den physikalischen und biochemischen Charakteristika der Wirkstoffe insbesondere in Hinblick auf ihre biologische Verfügbarkeit und systemische Wirkungsweise, die es hierbei zu beachten gilt.

Insbesondere Darreichungsformen für die äußere Anwendung sowie orale Applikationsformen sind hier für die Bereitstellung solcher stabilen hochpotonten Wirkstoffe von besonderem Interesse. Dabei sind für derartige Applikationen besonders geeignete und bevorzugte Darreichungsformen solche, die in wässrigen und/oder wasserhaltigen Formulierungen bzw. Umgebungen einsetzbar sind und in solchen wässrigen Milieus schnell-löslich sind. Dies ist insbesondere bei Wirkstoffsystemen für die orale Applikation von Bedeutung.

Es wurden bisher diverse Methoden, Darreichungsformen und Applikationssysteme entwickelt, um solche instabilen, leicht zersetzlichen und/oder leicht abbaubaren Substanzen zu stabilisieren und auch langfristig in kosmetischen und/oder pharmazeutischen Zusammensetzungen für die äußere oder auch orale Applikation verfügbar zu halten. Insbesondere zu nennen sind hier Methoden zur Verkapselung von Wirkstoffen, z.B. in Liposomen, die Verwendung spezieller Emulsionstechniken oder stabilisierende Lösungsmittel oder auch die Bereitstellung instabiler Wirkstoffe in Verabreichungssystemen, die eine stabile Derivatform oder Wirkstoffvorstufe sowie ein Agens zur Umsetzung des Wirkstoffs aus dem Precursor enthalten.

Nachteilig an diesen Methoden ist die begrenzte Beladungsdichte, die aufwendige Herstellung, der Kontakt wasserempfindlicher, wasserlöslicher oder wasserunlöslicher Wirkstoffe mit dem Lösungsmittel Wasser während der Lagerdauer sowie die zumeist unzureichende und ungenügend zuverlässige und schlecht reproduzierbare Freisetzungskinetik der stabilisierten Wirkstoffe aus den Zusammensetzungen.

Für hochkonzentrierte Wirkstoff-Verabreichungsformen besteht außerdem ein besonderes Interesse an sogenannten single-dose-unit Anwendungsformen, was eine einfache und zielgenaue Dosis-Applikation für den Endanwender ermöglicht. Als single-dose-unit Anwendungsformen werden hier Applikationssysteme verstanden, die im Gegensatz zu Pulvern oder Granulaten pro Anwendungseinheit die gewünschte und notwendige Wirkstoffmenge in einer einzigen Applikationseinheit enthalten wie beispielsweise Tabletten oder Kapseln, ohne dabei jedoch die Nachteile der schlechten Löslichkeit oder der fehlenden Eignung für die äußerliche Applikation mit sich zu führen.

Somit werden solche leichtlöslichen, durch Gefriertrocknung feuchtigkeitsstabilisierten single-dose-unit Applikationsformen für die orale und/oder äußerliche Anwendung von instabilen Wirkstoffen vermehrt in Form größer formatiger Ausgestaltungen interessant, insbesondere wenn hohe Mengen von Wirkstoffen verabreicht werden sollen. Dabei besteht die besondere Herausforderung in der Regel darin, hohe Wirkstoffgehalte in schnell- und möglichst vollständig bzw. rückstandslos löslicher Form bereitzustellen, wobei ein möglichst hoher Wirkstoffgehalt bei einem möglichst geringen Träger- oder Hilfsstoffanteil in der Zusammensetzung wünschenswert ist, da wie bereits eingehend dargestellt mit zunehmendem Träger- bzw. Hilfsstoffanteil, die rückstandslose und schnelle, vollständige Auflösung der Zubereitungen abnimmt.

Es besteht also die Notwendigkeit, gut dosierbare, großformatige single-dose-unit Applikationsformen mit hoher Wirkstoff-Beladung, insbesondere Beladung mit instabilen Wirkstoffen und möglichst geringem Anteil an unlöslichem, quellbarem Trägerstoff und somit schnellstmöglicher und vollständiger Löslichkeit und höchstmöglicher mechanischer Stabilität für die kosmetische und pharmazeutische äußerliche und orale Verwendung bereitzustellen.

Die Aufgabe der vorliegenden Erfindung bestand somit darin, eine Zusammensetzung zur Verfügung zu stellen, in der extrem hohe Wirkstoffmengen, besonders instabile Wirkstoffe, langfristig stabilisiert und bei der Anwendung schnell, effizient, spezifisch und hochaktiv freigesetzt und appliziert werden können, wobei die Stabilisierung der Wirkstoffe vorzugsweise durch eine Gefriertrocknung der Wirkstoff-Zusammensetzung erreicht werden sollte. Des weiteren bestand die Aufgabe darin, diese stabilen Wirkstoff-Zusammensetzungen so auszugestalten, dass sie eine hohe mechanische Festigkeit und eine ausreichende Größe aufweisen, um insbesondere zur kosmetischen oder pharmazeutischen Applikation in Form von sogenannten single-dose-units oder Einzeldosisanwendung verwendet werden zu können. Dabei sollen die Zusammensetzungen gleichermaßen für die äußerliche Anwendung sowie für eine orale oder perorale Applikation geeignet sein. Es war ferner Aufgabe der vorliegenden Erfindung eine Möglichkeit für diese mechanische Stabilisierung zu finden, die sich nicht nachteilig auf das Auflösungsverhalten der gefriergetrockneten Zusammensetzungen auswirkt und eine hohe Auflösungsgeschwindigkeit des gefriergetrockneten Endproduktes gewährleistet, insbesondere indem der Anteil an stabilisierenden, strukturgebenden Gerüstbildnern soweit wie möglich reduziert und idealerweise vollständig vermieden wird.

Überraschend wurde gefunden, dass ausgehend von der DE 10248314 auch solche stabilen, großformatigen wirkstoffbeladenen Formkörper hergestellt werden können, die Wirkstoffmengen enthalten, die mit $\geq$ 50 Gew.-% Wirkstoffgehalt und < 10 Gew.-% Gerüstbildneranteil außerhalb der in der DE 10248314 offenbarten Mengen liegen, indem die damit einhergehenden mechanischen Stabilitätsprobleme durch eine Beschichtung der Formkörper kompensiert werden. Dabei können überraschend insbesondere auch instabile Wirkstoffe in großen Mengen eingebracht werden, ohne dass durch hohe Gerüstbildneranteile die notwendige Stabilität erreicht, aber die Auflösungsgeschwindigkeit verringert wird.

Durch Wahl eines geeigneten filmbildenden hydrophilen Coatings und Aufbringen dieses Coatings auf den gefrorenen Formkörper und anschließende Gefriertrocknung des beschichteten gefrorenen Formkörpers ist es überraschend gelungen, einen gefriergetrockneten, mechanisch stabilen hochkonzentrierten Wirkstoff-Formkörper zu erhalten, der darüber hinaus aufgrund des erfindungsgemäß äußerst niedrigen Gerüstbildneranteils hinsichtlich seines Löslichkeitsverhaltens gegenüber bereits bekannten Systemen wie z. B. den in der DE 10248314 beschriebenen noch einmal deutlich verbessert werden konnte.

Weder die DE 10248314 noch eines der anderen hier diskutierten Dokumente offenbart feste gefriergetrocknete Formkörper mit derart hohem Wirkstoffgehalt und mit einer Beschichtung eines Filmbildners, die derart gute Eigenschaften hinsichtlich mechanischer Stabilität, Auflösungsverhalten und Größe für die Anwendung in der kosmetischen und pharmazeutischen Einzeldosis-Anwendung aufweist.

Die Erfindung stellt somit gefriergetrocknete Formkörper bereit, die einen oder mehrere Wirkstoffe und gegebenenfalls einen oder mehrere Gerüstbildner, gegebenenfalls einen oder mehrere Hilfsstoffe, sowie eine Beschichtung, enthaltend mindestens einen Filmbildner, enthalten.

Die Erfindung stellt darüber hinaus auch ein Verfahren zur Herstellung solcher gefriergetrockneter Formkörper, die Kombination solcher gefriergetrockneter Formkörper in Kit-of-parts Anordnungen zusammen mit wässrigen Lösungen sowie die Verwendung der gefriergetrockneten Formkörper und der Kit-of-parts Kombinationen zur pharmazeutischen und kosmetischen Anwendung bereit.

Unter einem Formkörper im Sinne der Erfindung versteht man einen regelmäßig geformten geometrischen Körper, z.B. insbesondere Kugeln, Quader, Pyramiden, Sterne aber auch natürlichen Formen nachgebildete Formkörper wie z.B. solche in der Form von Tieren, wie z.B. Meerestieren, wie z.B. Seesterne, Meeresfrüchte, wie Muscheln, etc. Pflanzen und Pflanzenteilen, wie Blätter etc. Nach dem weiter unten beschriebenen Verfahren zur Herstellung der erfindungsgemäß verwendeten Formkörper sind alle diese Formen zugänglich. Erfindungsgemäß bevorzugt sind einheitliche regelmäßige sphärische Formen wie insbesondere eine Kugelgeometrie, da diese sich insbesondere bei der Beschichtung mit dem filmbildenden Coating als besonders vorteilhaft hinsichtlich homogener Beschichtung erweist und außerdem aufgrund des besonders günstigen Oberfläche/Volumen Verhältnisses besonders gut zu mechanisch stabilen Formkörpern verarbeiten lässt. Die Sublimationsstrecke durch das schon trockene Produkt ist in sphärischen oder kugelförmigen Formkörpern nach allen Seiten hin symmetrisch und klein, was den Dampftransport durch das schon trockene Gut im Rahmen des Gefriertrocknungsprozesse erleichtert.

Erfindungsgemäß ist auch eine Mehrzahl der genannten Formkörper in einem Behältnis umfasst. Auch kann es sich um Mischungen von Formkörpern verschiedener Geometrien oder unterschiedlicher Größen handeln. Die Formkörper können einzeln abgepackt sein, bevorzugt liegt jedoch insbesondere in der kosmetischen Anwendung eine Mehrzahl der Formkörper nebeneinander in Kontakt in einem Behältnis vor.

Die Volumina der verwendeten Formkörper sind aufgrund des Verfahrens ihrer Herstellung an sich nicht beschränkt. Zweckmäßig liegen die Volumina bevorzugt bei mindestens etwa 0,1 $cm^3$, bevorzugt 0,3 $cm^3$, bevorzugter mindestens etwa 0,5 $cm^3$, noch bevorzugter mindestens etwa 0,6 $cm^3$. Nach oben werden die verwendeten Volumina zweckmäßig auf bis zu etwa 6 $cm^3$, bevorzugt bis zu etwa 5 $cm^3$, bevorzugter bis zu etwa 4 $cm^3$ beschränkt. Die Größe der Formkörper wird unter anderem durch die gewünschte Applikationsform oder den Ort der äußeren Anwendung der Formkörper bestimmt. So ermöglicht bei der äußeren kosmetischen oder pharmazeutischen Verwendung die Applikation auf größeren Körperflächen oder den Haaren (z.B. der direkte Auftrag der angefeuchteten Formkörper auf dem Rücken etc., oder der Einsatz als Badezusatz) den Einsatz größerer Formkörper, wohingegen bei der Anwendung auf kleineren Körperpartien (z.B. Wange etc.) kleinere Formkörper bevorzugt sind.

Auch bei der Herstellung von Formkörpern zur oralen Applikation kann die Größe angepasst werden. So ist beispielsweise denkbar, die Formkörpergröße auf die entsprechende Zielgruppe abzustimmen, wobei denkbar ist, älteren

Anwendern größere Formkörper mit besserer Handhabbarkeit und leichterem Handling anzubieten und z. B. jüngeren Anwendern oder Kindern solche, die in einem abgestimmten Verhältnis zu ihrer Körpergröße und der altersbedingt zu erwartenden Compliance bei der Anwendung stehen.

Der Durchmesser eines Formkörpers (maximaler Abstand zwischen zwei Punkten in einem Formkörper jedweder Geometrie) liegt zweckmäßig bei mindestens etwa 3 mm, bevorzugt mindestens etwa 5 mm, bevorzugter mindestens etwa 7 mm, noch bevorzugter mindestens etwa 8 mm bis hin zu zweckmäßig etwa 60 mm, bevorzugt etwa 50 mm, bevorzugter etwa 40 mm, noch bevorzugter etwa 20 mm. Ein besonders bevorzugter Formkörper weist aus besagten Gründen eine im wesentlichen kugelförmige Geometrie auf, wobei der Durchmesser der Kugel zwischen 3 bis 30 mm, bevorzugt zwischen 5 und 20 mm, bevorzugter zwischen 7 und 15 mm, noch bevorzugter zwischen 8 und 14 mm liegt. Besonders bevorzugt sind Formkörper in Form einer Kugel mit einem Durchmesser von mindestens 6 mm.

Die erfindungsgemäßen gefriergetrockneten Formkörper enthalten mindestens einen oder mehrere Wirkstoffe, bevorzugt mindestens einen Wirkstoff in einer Menge von ≥50 Gew.-% bezogen auf die beschichtete gefriergetrocknete Gesamtzusammensetzung. Wirkstoffe schließen insbesondere kosmetische oder therapeutische bzw. pharmazeutische, für die äußere Anwendung sowie für die orale oder perorale Applikation geeignete Wirkstoffe ein. Bevorzugt enthält der erfindungsgemäß verwendete Formkörper mindestens einen kosmetischen und/oder pharmazeutischen Wirkstoff. Dementsprechend handelt es sich bei den erfindungsgemäßen gefriergetrockneten Formkörpern bevorzugt um kosmetische oder therapeutische Mittel.

Kosmetische Formkörper bzw. unter Verwendung kosmetischer Wirkstoffe hergestellte Formkörper im Sinne der Erfindung sind im wesentlichen Mittel im Sinne des Lebens-mittel-, Bedarfsgegenstände- und Futtermittelgesetzbuches (LFGB), d.h., Stoffe oder Zubereitungen aus Stoffen, die dazu bestimmt sind, äußerlich am Menschen zur Reinigung, Pflege, oder zur Beeinflussung des Aussehens oder des Körpergeruchs, oder zur Vermittlung von Geruchseindrücken angewendet zu werden, es sei, denn, dass sie überwiegend dazu bestimmt sind, Krankheiten, Leiden, Körperschäden oder krankhafte Beschwerden zu lindern oder zu beseitigen. In diesem Sinne handelt es sich bei den erfindungsgemäß verwendeten kosmetischen Formkörpern beispielweise um Badepräparate, Hautwasch- und reinigungsmittel, Hautpflegemittel, insbesondere Gesichtshautpflegemittel, Augenkosmetika, Lippenpflegemittel, Nagelpflegemittel, Fußpflegemittel, Haarpflegemittel, insbesondere Haarwaschmittel, Haarkonditionierungsmittel, Haarweichspüler etc., Lichtschutzmittel, Hautbräunungs- und -aufhellungsmittel, Depigmentierungsmittel, Deodorants, Antihydrotika, Haarentfernungsmittel, Insektenrepellents etc. oder derartige Mittel in Kombination.

Beispiele kosmetisch gegebenenfalls auch z.B. dermatologischer, therapeutisch wirksamer Verbindungen schließen ein: Antiaknemittel, antimikrobielle Mittel, Antitranspirationsmittel, adstringierende Mittel, desodorierende Mittel, Enthaarungsmittel, Konditionierungsmittel für die Haut, hautglättende Mittel, Mittel zur Steigerung der Hauthydratation wie z. B. Glycerin oder Harnstoff, Sonnenschutzmittel, Keratolytika, Radikalfänger für freie Radikale, Antiseborrhöika, Antischuppenmittel, antiseptische Wirkstoffe, Wirkstoffe zur Behandlung der Anzeichen der Hautalterung und/oder Mittel, die die Differenzierung und/oder Proliferation und/oder Pigmentierung der Haut modulieren, Vitamine wie Vitamin C (Ascorbinsäure) und ihre Derivate, Wirkstoffe mit reizender Nebenwirkung, wie alpha-Hydroxysäuren, β-Hydroxysäuren, alpha-Ketosäuren, β-Ketosäuren, Retinoide (Retinol, Retinal, Retin-Säure) Anthralinen (Dioxyanthranol), Anthranoide, Peroxide (insbesondere Benzoylperoxid), Minoxidil, Lithiumsalze, Antimetabolite, Vitamin D und seine Derivate; Katechine, Flavonoide, Ceramide, mehrfach ungesättigte Fettsäuren, essentielle Fettsäuren (z.B. gamma-Linolensäure), proteinogene Wirkstoffe mit einem Molekulargewicht von weniger als 1000 kDa wie z.B. Enzyme, Coenzyme, Peptide, Enzymhemmstoffe, hydratisierende Mittel, hautberuhigende Mittel, Detergentien oder schaumbildende Mittel, und anorganische oder synthetische mattierende Füllstoffe, oder dekorative Wirkstoffe wie Pigmente oder Farbstoffe und -partikel für Foundations, Make-up Formulierungen, und andere Mittel zur kosmetischen Verschönerung und farblichen Gestaltung von Augen-, Lippen-, Gesicht etc. sowie abrasive Mittel.

Weiterhin können Pflanzenwirkstoffextrakte bzw. daraus gewonnene Auszüge oder Einzelstoffe erwähnt werden. Allgemein wird der Pflanzenwirkstoffextrakt in der Regel ausgewählt aus der Gruppe bestehend aus festen Pflanzenextrakten, flüssigen Pflanzenextrakten, hydrophilen Pflanzenextrakten, lipophilen Pflanzenextrakten, einzelnen Pflanzeninhaltsstoffen; sowie deren Mischungen, wie Flavonoide und ihre Aglyka: Rutin, Quercetin, Diosmin, Hyperosid, (Neo)hesperidin, Hesperitin, Ginkgo Biloba (z.B. Ginkoflavonglykoside), Crataegus-Extrakt (z.B. oligomere Procyanidine), Buchweizen (z.B. Rutin), Sophora japonica (z.B. Rutin), Birkenblätter (z.B. Quercetinglykoside, Hyperosid und Rutin), Holunderblüten (z.B. Rutin), Lindenblüten (z.B. ätherisches Öl mit Quercetin und Farnesol), Johanniskautöl, (z.B. Olivenölauszug), Calendula, Arnika (z.B. ölige Auszüge der Blüten mit ätherischem Öl, polare Auszüge mit Flavonoiden), Melisse (z.B. Flavone, ätherisches Öl); Immunstimulantien: Echinacea purpurea (z.B.alkoholische Auszüge, Frischpflanzensaft, Preßsaft), Eleutherokokkus senticosus; Alkaloide: Rauwolfia (z.B. Prajmalin),Immergrün (z.B.Vincamin); weitere Phytopharmaka: Aloe, Roßkastanie (z.B. Aescin), Knoblauch (z.B. Knoblauchöl), Ananas (z.B. Bromelaine), Ginseng (z.B. Ginsenoside), Mariendistelfrüchte (z.B. auf Silymarin standardisierter Extrakt), Mäusedornwurzel (z.B. Ruscogenin), Baldrian (z.B. Valepotriate, Tct. Valerianae), Kava-Kava (z.B. Kavalactone), Hopfenblüten (z.B. Hopfenbitterstoffe), Extr. Passi-florae, Enzian (z.B. ethanol. Extrakt), anthrachinonhaltige Drogenauszüge, z.B. aloinhaltiger Aloe Vera-Saft, Pollenextrakt, Algenextrakte, Süßholzwurzelextrakte, Palmenextrakt, Galphimia (z.B.Urtinktur), Mistel (z.B.

wässrig-ethanol. Auszug), Phytosterole (z.B. beta-Sitosterin), Wollblumen (z.B. wäßrig-alkohol. Extrakt), Drosera (z.B. Likörweinextrakt), Sanddornfrüchte (z.B. daraus gewonnener Saft oder Sanddornöl), Eibischwurzel, Primelwurzelextrakt, Frischpflanzenextrakte aus Malve, Beinwell, Efeu, Schachtelhalm, Schafgarbe, Spitzwegerich (z.B. Preßsaft), Brennnessel, Schöllkraut, Petersilie; Pflanzenextrakte aus Norolaena lobata, Tagetes lucida, Teeoma siems, Momordica charantia, und Aloe Vera Extrakte.

Bevorzugte kosmetische Wirkstoffe sind solche, die eine hohe Instabilität gegen Abbau oder Zersetzung wie insbesondere bedingt durch Feuchtigkeitszufuhr aufweisen, sowie solche Wirkstoffe, die in wässriger Lösung einen sauren pH-Wert aufgrund saurer Gruppen im Wirkstoff erzeugen, sogenannte saure Wirkstoffe, die selbst durch Gefriertrocknungsverfahren bisher nicht oder nur in sehr geringen Konzentrationen bzw. unter sehr hohem Kostenaufwand durch eine Prozessführung bei niedrigsten Temperaturen mit sehr langen Trocknungszeiten in zufriedenstellendem Maße in stabile Formen überführt werden konnten.

Ein besonders bevorzugter insbesondere in der Kosmetik verbreiteter Wirkstoff aus der Gruppe dieser instabilen, sauren Wirkstoffe ist die Ascorbinsäure oder Vitamin C und ihre Derivate oder auch Vitamin A und Derivate davon.

Derivate der Ascorbinsäure sind insbesondere Glycoside wie Ascorbylglucosid, oder Ester der Ascorbinsäure wie Natrium- oder Magnesium-Ascorbylphosphat oder Ascorbylpalmitat und -stearat, sowie beispielsweise L-Ascorbinsäurephosphatester, Alkalimetallsalze wie Natrium- und Kaliumsalze von L-Ascorbinsäurephosphatestern; Erdalkalimetallsalze wie Magnesium- und Calciumsalze von L-Ascorbinsäurephosphatestern; trivalente Metallsalze wie Aluminiumsalze von L-Ascorbinsäurephosphatestern; Alkalimetallsalze von L-Ascorbinsäuresulfatestern wie Natrium- und Kaliumsalze von L-Ascorbinsäuresulfatestern; Erdalkalimetallsalze wie Magnesium- und Calciumsalze von L-Ascorbinsäuresulfatestern; trivalente Metallsalze wie Aluminiumsalze von L-Ascorbinsäuresulfatestern; Alkalimetallsalze wie Natrium- und Kaliumsalze von L-Ascorbinsäureestern; Erdalkalimetallsalze wie Magnesium- und Calciumsalze von L-Ascorbinsäureestern; und trivalente Metallsalze wie Aluminiumsalze von L-Ascorbinsäureestern.

Im Unterschied zu den vorstehend beschriebenen im wesentlichen in der Kosmetik verwendeten Formkörpern handelt es sich bei den therapeutisch verwendeten Formkörpern (Arzneimittel) um solche, die mindestens einen pharmazeutischen bzw. therapeutischen insbesondere auch dermatologischen Wirkstoff enthalten und die im Sinne des Arzneimittelgesetzes unter anderem dazu bestimmt sind, Krankheiten, Leiden, Körperschäden oder krankhafte Beschwerden zu heilen, zu lindern oder zu verhüten. Solche Mittel bzw. Wirkstoffe sind sowohl für die äußere Anwendung als auch für die orale oder perorale Applikation bestimmt.

Bei Wirkstoffen für die äußere Anwendung handelt es sich insbesondere um hautaktive aber auch um transdermale Wirkstoffe. Sie schließen beispielsweise ein: Mittel zur Behandlung von Hautkrankheiten, äußerlich anwendbare Analgetika, z. B. Dextropropoxyphen, Pentazocin, Pethidin, Buprenorphin; Antirheumatika/Antiphlogistika (NSAR), z. B. Indometacin, Diclofenac, Naproxen, Ketoprofen, Ibuprofen, Flurbiprofen, Salicylsäure und - derivate wie Acetylsalicylsäure, Oxicame; Steroidhormone, z. B. Betamethason, Dexamethason, Methylprednisolon, Ethinylestradiol, Medroergotamin, Dihydroergotoxin; Gichtmittel, z. B. Benzbromaron, Allopurinol; Dermatika Externa, einschließlich antibakterielle Mittel wie z.B. kolloidales Silber oder Silbersalze, Antimykotika, antivirale Wirkstoffe, entzündungshemmende Wirkstoffe, juckreizstillenden Wirkstoffe, anästhesierende Wirkstoffe, z. B. Benzocain, Corticoide, Aknemittel, antiparasitäre Wirkstoffe; äußerlich anwendbare Hormone; Venentherapeutika; Immunsuppresiva etc. alle für die äußerliche Anwendung.

Bevorzugte therapeutische Mittel für die äußere Anwendung sind Analgetika, z.B. Immunsuppressiva, Hormone, Mittel zur Behandlung von Hautkrankheiten, wie der Neurodermitis, der atopischen Dermatitis etc., und Anti-Herpesmittel.

Therapeutische Wirkstoffe für die orale oder perorale Applikation können aus gewählt sein aus der Gruppe der Antihistaminika, der Antibiotika, der Peptidarzneistoffe, Antimykotika, Bronchialtherapeutika wie Antiasthmatika, Antitussiva, Mucolytica etc., Antidiabetica wie z. B. Glibenclamid, Hormone, Steroidhormone wie Dexamethason, Herzglycoside wie Digitoxin, Herz- und Kreislauftherapeutika wie z. B. Beta-Blocker, Antiarrhythmika, Antihypertonika, Calcium-Antagonisten etc., Psychopharmaka und Antidepressiva wie z. B. trizyklische Antidepressiva (NSMRI), Serotonin-Wiederaufnahme-Hommer (SSRI), Noradrenalin-Wiederaufnahme-Hemmer (NRI), Serotonin-Noradrenalin-Wiederaufnahme-Hemmer (SNRI), Monoaminooxidase-Hemmer (MAO-Hemmer) etc., Neuroleptika, Antikonvulsiva oder Antiepileptika, Hypnotika, Sedativa, Anästhetika, Magen-, Darmtherapeutika, Lipidsenker, Analgetika wie z. B. Antimigränemittel, Paracetamol, Salicylsäure und -derivate wie Acetylsalicylsäure, Diclophenac, Ibuprofen, Ketoprofen, Naproxen etc. , Antiphlogistika, Vasodilatatoren, Diuretica, Gichtmittel, Zytostatika, Muskelrelaxantien, Pflanzenextrakte, Provitamine wie z. B. Beta-Carotin, Vitamine wie z. B. Vitamin C (Ascorbinsäure), A, B, E etc., Kieselerde, Mineralstoffe und Spurenelemente wie z. B. Kalium, Magnesium, Calcium, Selen, Iod etc., Diätsupplemente und Nahrungsergänzungsmittel usw. alle für die orale oder perorale Applikation.

Ein besonders bevorzugter pharmazeutischer Wirkstoff, der sowohl für die äußerliche als auch die orale oder perorale Applikation verwendet und ausgewählt ist aus der Gruppe der instabilen, sauren Substanzen ist die Salicylsäure-und ihre Derivate wie Acetylsalicylsäure (ASS). Weitere bevorzugte instabile, saure und gefrierpunkterniedrigende therapeutische Wirkstoffe sind Clofibrinsäure, Ibuprofen, Gemfibrozil, Fenoprofen, Naproxen, Ketoprofen, Indomethacin,

Bezafibrat, Tolfenaminsäure, Diclofenac, Meclofenaminsäure, Paracetamol, Acitretin, Acrivastin, Azelainsäure, Cromolyn, Ethacrynsäure, Furosemid, Penicillin und Derivate davon, Risedronsäure und Derivate davon, Liponsäure, und Ursodiol.

Die erfindungsgemäßen gefriergetrockneten Formkörper Weisen bevorzugt einen Wirkstoffanteil ≥ 50 Gew. %, bevorzugt ≥ 75 Gew. %, bevorzugter ≥ 80 Gew. %, noch bevorzugter ≥ 90 Gew. % jeweils bezogen auf die Gesamtzusammensetzung des gefriergetrockneten, beschichteten Formkörpers auf.

Dabei handelt es sich insbesondere um solche Wirkstoffe, die ausgewählt sind aus der Gruppe der sauren Wirkstoffe, also um Wirkstoffe, die in wässriger Lösung einen sauren pH-Wert aufgrund saurer Gruppen im Wirkstoff erzeugen. Bei solchen sauren Wirkstoffen handelt es sich speziell um Wirkstoffe, deren 1 gewichtsprozentige Lösung oder Suspension in Wasser bei 20 °C einen pH Wert < 7 aufweist, bzw. um solche Wirkstoffe, die einen pKs-Wert bei 25 °C von ≤ 7 aufweisen.

Der pKs-Wert bezeichnet dabei den negativen dekadischen Logarithmus der Säurekonstante Ks. Die Säurekonstante Ks ist eine Stoffkonstante und gibt Aufschluss darüber, in welchem Maß ein Stoff (HA) in einer Gleichgewichtsreaktion mit Wasser unter Proteolyse reagiert:

$$HA + H_2O \rightleftharpoons H_3O^+ + A^-.$$

Dabei steht HA für eine Brønsted-Säure (nach Brønsted), die ein Proton $H^+$ an ein Lösungsmittel wie Wasser abgeben kann, wobei ein Anion $A^-$ zurückbleibt. Allgemeiner gilt die Br∅nsted'sche Definition auch für nichtwässrige Systeme, hier gilt für ein beliebiges, protonierbares Lösungsmittel Y:

$$HA + Y \rightleftharpoons HY^+ + A^-.$$

Die Säurekonstante Ks bezeichnet dabei die Gleichgewichtskonstante dieser Reaktion und ist damit ein Maß für die Stärke einer Säure. Je stärker die Säure, desto mehr ist die Reaktion auf die rechte Seite verschoben. Somit ergibt sich, daß je kleiner der pKs-Wert, desto stärker die Säure.

Die Bestimmung des pKs-Wertes erfolgt über pH-Messung in einer sogenannten Halbtitration. Dabei wird eine Lösung der Säure bekannter Konzentration vorgelegt und der pH-Wert z.B. mittels pH-Messsonde gemessen. Daraufhin wird die Säure teilweise mit einer Maßlösung einer Base der gleichen Wertigkeit der vorgelegten Säure neutralisiert. Hierbei wird exakt die Hälfte der Stoffmenge der Säure aus der Vorlage zugesetzt. Nun wird wiederum der pH-Wert bestimmt. Es gilt:

$$pK_S = -lgK_S = -lg\frac{c[H^+] \cdot c[A^-]}{c[HA]}$$

Da nach Zugabe der halben Stoffmenge gilt, dass c[A - ] = c[HA] , gilt für den sogenannten Halbtitrationspunkt, dass pKs = pH.

Derartige saure Wirkstoffe verfügen über eine pH-Wert abhängige hohe Dissoziationsneigung, wodurch in neutralen bis alkalischen pH-Wertebereichen der Wirkstoff in dissoziierter Form und somit in einer hohen Ionenkonzentration vorliegt. Eine derartig erhöhte Ionenkonzentration führt dann zu einer gefrierpunkterniedrigenden Wirkung, die sich nachteilig auf den Gefriertrocknungsprozess auswirkt. Zusammensetzungen, die insbesondere hohe Mengen gefrierpunkterniedrigender Substanzen enthalten. Durch die gefrierpunkterniedrigende Wirkung solcher dissoziierten Wirkstoffe bilden sich im gefrorenen Formkörper in der Regel große Eiskristalle mit einem hohen Anteil nicht einfrierbaren Wassers mit hohen Aktivstoffkonzentrationen, die zu einem partiellen Strukturkollaps des gefriergetrockneten Endproduktes, zu einem sogenannten Antauen des Formkörpers führen, weshalb solche sauren Wirkstoffe durch Gefriertrocknungsverfahren bisher nicht oder nur in sehr geringen Konzentrationen bzw. unter sehr hohem Kostenaufwand durch eine Prozessführung bei niedrigsten Temperaturen mit sehr langen Trocknungszeiten in zufriedenstallendem Maße in stabile gefriergetrocknete Formen überführt werden konnten.

In einer bevorzugten Ausführungsform enthalten die erfindungsgemäßen gefriergetrockneten Formkörper bezogen auf die Gesamtzusammensetzung des gefriergetrockneten beschichteten Formkörpers ≥ 50 Gew. % eines Wirkstoffs aus der Gruppe der Ascorbinsäure und ihrer Derivate.

In einer weiteren bevorzugten Ausführungsform enthalten die erfindungsgemäßen gefriergetrockneten Formkörper bezogen auf die Gesamtzusammensetzung des gefriergetrockneten Formkörpers ≥ 50 Gew.% eines Wirkstoffs aus der Gruppe der Salicylsäure und deren Derivate, bevorzugt aus der Gruppe der Acetylsalicylsäure und ihrer Derivate.

Die erfindungsgemäßen Formkörper enthalten vorzugsweise nur sehr geringe Mengen bis maximal 25 Gew.-%, bevorzugt bis maximal 10 Gew.-% eines oder mehrerer Gerüstbildner bezogen auf die Gesamtzusammensetzung des gefriergetrockneten, beschichteten Formkörpers. Bei solchen Gerüstbildnern handelt es sich im allgemeinen um sogenannte Hydrokolloide, d.h. (teilweise) wasserlösliche/-quellbare, natürliche oder synthetische Polymere, die in wässrigen Systemen Gele bzw. viskose Lösungen bilden. Die Gerüstbildner werden zweckmäßig ausgewählt aus Polysacchariden, Mucopolysacchariden, tierischen Polyaminosacchariden wie Chitin oder dessen Derivate, insbesondere Chitosan oder aus den Glucosaminoglykanen sowie den synthetischen Polymeren. Bevorzugt wird der Gerüstbildner aus der Gruppe der Polysaccharide ausgewählt. Polysaccharide schließen beispielsweise Homoglykane oder Heteroglykane ein, wie zum Beispiel Alginate, besonders Natriumalginat, Carrageen, Pektine, Tragant, Guar-Gummi, Pullulan, Trehalose, Johannisbrotkernmehl, Agar-Agar, Gummi-Arabikum, Xanthan, natürliche und modifizierte Stärken wie z.B. kationisierte Stärkederivate, Dextrane, Dextrin, Maltodextrine, Glucane, wie β-1,3-Glucan, β-1,4-Glucan, wie Cellulose, Mucopolysaccharide, wie Hyaluronsäure etc., sowie tierische Polyaminosaccharide wie Chitin oder dessen Derivate wie insbesondere Chitosan. Synthetische Polymere schließen beispielsweise ein: Celluloseether, Polyvinylalkohol, Polyvinylpyrrolidon, synthetische Cellulosederivate, wie Mothylcellulose, Carboxycellulose, Carboxymethylcellulose, kationisierte Carboxymethylcellulose, Celluloseester, Cellulosesether wie Hydroxypropylcellulose, Polyacrylsäure, Polymethacrylsäure, Poly(methylmethacrylat) (PMMA), Polymethacrylat (PMA), Polyethylenglykole etc. Es können auch Mischungen mehrerer Gerüstbildner verwendet werden. Erfindungsgemäß besonders bevorzugt sind Alginate, wie insbesondere Natrium-Alginat. Bevorzugt sind hydrophile und leicht wasserlösliche Gerüstbildner, insbesondere calciumfreie Natrium-Alginate, (Natrium-Alginat mit einem Calciumgehalt < 3 Gew.-%, bevorzugter < 2 Gew.-%, noch bevorzugten < 1,5 Gew.-%), Cellulosen wie z.B Carboxymethylcellulose, Hyaluronsäure sowie Chitosan oder kationisch modifizierte Stärke oder kationisch modifizierte Carboxymethylcellulose.

In einer bevorzugten Ausführungsform enthalten die erfindungsgemäßen gefriergetrockneten Formkörper mindestens einen Gerüstbildner, der ausgewählt ist aus der Gruppe der kationischen Gerüstbildner. Darunter versteht man allgemein solche Gerüstbildner, die unter physiologischen Umgebungsbedingungen (Raumtemperatur, neutraler pH-Wertebereich, wässriges Milieu) mehr positive als negative Ladungen auf ihrer Oberfläche aufweisen.

Insbesondere umfassen kationisch modifizierte Polymere solche, worin mindestens eine Seitengruppe des Polymergerüstes durch kationische Gruppen substituiert ist. Erfindungsgemäß sind dabei insbesondere solche kationisch modifizierten Polymere bevorzugt, die einen Substitutionsgrad (Sga) ≥ 1 % aufweisen. Dabei kann die Bestimmung des Substitutionsgrades in Abhängigkeit der Art der kationischen Gruppe im modifizierten Polymer nach dem Fachmann bekannten und jeweils geeigneten Standarduntersuchungsmethoden erfolgen.

Im allgemeinen fallen unter den Begriff der kationischen Polymere bzw. Gerüstbildner insbesondere modifizierte Chitinderivate wie insbesondere Chitosan, aber auch andere chemisch modifizierte Biopolymere, wie kationisierte Cellulosen wie z.B. kationisierte Carboxymethylcellulose oder kationisierte Stärken.

Kationische Biopolymere auf Polysaccharid-Basis wie z.B. Cellulosen oder Stärke umfassen dabei solche, bei denen einige Hydroxygruppen in den Polymerseitenketten mit kationischen Gruppen oder Gruppen, die in saurem Medium durch Protonierung in kationische Gruppen umgewandelt werden können, verethert sind. Diese Substituenten können beispielsweise aus tertiären Aminogruppen oder quaternären Ammoniumsalzen oder auch aus Sulfoniumgruppen oder Phosphoniumgruppen bestehen.

Insbesondere bei Verwendung von Wirkstoffen aus der Gruppe der sauren Wirkstoffe wie insbesondere Ascorbinsäure und deren Derivaten oder Acetylsalicylsäure und deren Derivaten ist die Verwendung von leichtlöslichen kationischen Gerüstbildnern wie Chitosan oder kationisierten Stärkederivaten oder kationisierten Cellulosederivaten (z.B. Carboxymethylcellulose) gegebenenfalls auch in Mischung mit weiteren der vorstehend genannten Gerüstbildner besonders bevorzugt.

In einer bevorzugten Ausführungsform ist die Verwendung von Gerüstbildnern aus der Gruppe der Proteine ausgenommen.

Die Verwendung solcher hydrophiler, leicht wasserlöslicher Gerüstbildner ist zum einen herstellungsbedingt bevorzugt, zum anderen führt die Anwendung solcher hydrophiler Gerüstbildner zu einer leichten Löslichkeit der Formulierung z. B. im Mund durch den Speichel bzw. bei Zusatz von Wasser oder wässrigen Lösungen zu einer hohen Zerfalls- bzw. Auflösungsgeschwindigkeit und damit zu einer leichten Verteilbarkeit auf der Haut. Insbesondere die Verwendung von leicht löslichen Gerüstbildnern in den bevorzugten geringen Gehalten von ≤ 10 Gew.-% bezogen auf die Gesamtzusammensetzung des gefriergetrockneten, beschichteten Formkörpers kann zu einer größeren Auflösungsgeschwindigkeit der erfindungsgemäßen Formkörper führen. Besonders bevorzugte Ausführungsformen kommen gänzlich ohne den Zusatz solcher gerüstbildender Strukturpolymere in der Wirkstoffzusammensetzung aus.

Dabei ist jedoch klarstellend zu unterscheiden zwischen solchen polymeren Gerüstbildnern, die in homogener

Verteilung mit der wirkstoffhaltigen Zusammensetzung, also im inneren Kernbereich des gefriergetrockneten Formkörpers vorliegen und solchen Polymeren, die die äußere Beschichtung bilden. Eine oben genannte besonders bevorzugte Ausführungsform, die ohne gerüstbildende Strukturpolymere auskommt, weist erfindungsgemäß dennoch eine äußere Beschichtung eines filmbildenden Polymers, wie nachstehend definiert, auf. Das Fehlen von gerüstbildenden Strukturpolymeren in solchen bevorzugten Ausführungsformen bezieht sich hierbei auf die den inneren Bereich des Formkörpers bildende Wirkstoffzusammensetzung.

Bisher war der Einsatz geringer Mengen von gerüstbildenden Substanzen in den erfindungsgemäßen Wirkstoff-Formkörpern gegenüber beispielsweise reinen, getrockneten, Zusatzstoff-freien Wirkstoffen zwingend notwendig, um den Wirkstoff unmittelbar in einer geeigneten mechanisch stabilen Darreichungsform bereitstellen zu können, was insbesondere in der äußerlichen Anwendung hinsichtlich Rppliziorbarkeit und Handhabbarkeit eine Rolle spielt. Zum Anderen sind die Gerüstbildner-Anteile maßgeblich zur Erzielung einer ausreichenden Stabilität der Wirkstoff-Formkörper.

Die erfindungsgemäß bevorzugt als Gerüstbildner verwendeten Polysaccharide weisen zweckmäßig durchschnittliche Molmassen von etwa $10^3$ bis zu etwa $10^8$, bevorzugt etwa $10^4$ bis $10^7$ auf.

Die erfindungsgemäßen gefriergetrockneten Formkörper zeichnen sich vorzugsweise dadurch aus, dass sie einen Gerüstbildneranteil ≤ 25 Gew. %, bevorzugt ≤ 10 Gew. %, bevorzugter ≤ 5 Gew. %, bezogen auf die Gesamtzusammensetzung des gefriergetrockneten beschichteten Formkörpers enthalten.

Die Gerüstbildner sind haut- und schleimhautverträglich und besitzen weder bei der äußerlichen noch bei der oralen oder peroralen Applikation ein toxikologisches Potential, insbesondere rufen sie keine Irritationswirkungen oder andere Unverträglichkeitsreaktionen hervor. Sie sind pharmakologisch unbedenklich und somit optimal als Trägermaterial für die erfindungsgemäßen kosmetischen und pharmazeutischen äußerlichen und oralen bzw. peroralen Verwendungen geeignet.

Auch die Gerüstbilder, insbesondere die Polysaccharide können gewisse therapeutische Wirkungen aufweisen. So wirkt der bevorzugt verwendete Gerüstbildner (Natrium-)Alginat im gewissen Ausmaß antiviral, er ist jedoch kein Wirkstoff im Sinne der Erfindung.

Die erfindungsgemäßen Formkörper enthalten weiterhin gegebenenfalls einen oder mehrere Hilfsstoffe. Hilfsstoffe schließen ein: Fettsubstanzen, wie Mineralöle, wie Paraffinöle oder Vaselineöle, Siliconöle, Pflanzenöle wie Kokosöl, Süßmandelöl, Aprikosenöl, Maisöl, Jojobaöl, Olivenöl, Avocadoöl, Sesamöl, Palmöl, Eukalyptusöl, Rosmarinöl, Lavendelöl, Kiefernöl, Thymianöl, Minzöl, Kardamonöl, Orangenblütenöl, Sojaöl, Kleieöl, Reisöl, Rapsöl und Rizinusöl, Weizenkeimöl und daraus isoliertes Vitamin E, Nachtkerzenöl, Pflanzenlecithine (z.B. Sojalecithin), aus Pflanzen isolierte Sphingolipide/Ceramide, tierische Öle oder Fette, wie Talg, Lanolin, Butteröl, Fettsäureester, Ester von Fettalkoholen wie Triglyceride und Wachse mit einem der Hauttemperatur entsprechenden Schmelzpunkt (tierische Wachse, wie Bienenwachs, Carnaubawachs und Candelillawachs, mineralische Wachse, wie mikrokristalline Wachse, und synthetische Wachse, wie Polyethylen- oder Silikonwachse), sowie sämtliche für kosmetische Zwecke geeigneten Öle (sogenannte kosmetische Öle), wie beispielsweise in der CTFA-Abhandlung, Cosmetic Ingredient Hand-book, 1. Auflg., 1988, The Cosmetic, Toiletry and Fragrance Association, Inc., Washington, erwähnt, pH-Einstellungsmittel, wie Puffersubstanzen, oberflächenaktive Mittel neben den oben erwähnten Waschtensiden, wie Dispergiermittel, Emulgatoren etc., Füllstoffe, Stabilisatoren, Cosolventien, pharmazeutisch und kosmetisch gebräuchliche oder sonstige Farbstoffe und Pigmente, insbesondere solche, die primär zur farblichen Gestaltung der Formkörper eingesetzt werden und nicht zur Applikation und farblichen Gestaltung des menschlichen Körpers, wie solche Pigmente und Farbstoffe wie die unter der Gruppe der Wirkstoffe aufgeführten dekorativen Farbstoffe, Konservierungsmittel, Weichmacher, Schmiermittel bzw. Gleitmittel, etc.

Ein besonders bevorzugter Hilfsstoff ist Neutralöl (Capryl/Capronsäure-Triglyceride).

Weitere erfindungsgemäß bevorzugte Hilfsstoffe sind Mannitol, sowie aus der Gruppe der pH-Einstellungsmittel Salzsäure und Natronlauge.

Die Hilfsstoffe können den erfindungsgemäßen Formkörpern in Mengen von bis zu 50 Gew.-% bezogen auf die Gesamtzusammensetzung zugesetzt werden.

Die erfindungsgemäßen Formkörper weisen bevorzugt einen Gesamtanteil an Wirk- und Hilfsstoffen von ≥ 50 Gew. %, bevorzugt ≥ 75 Gew. %, bevorzugter ≥ 80 Gew. %, noch bevorzugter ≥ 90 Gew. % bezogen auf die Gesamtzusammensetzung des gefriergetrockneten beschichteten Formkörpers auf.

Das Gewichtsverhältnis von Wirkstoffen zu Hilfsstoffen in den gefriergetrockneten beschichteten Formkörpern ist vorzugsweise ca. 10:1 bis 100:1 , bevorzugter ca. 20:1 bis 50:1 , wobei ein Gewichtsverhältnis von 20:1 besonders bevorzugt ist und wobei jeweils die Gesamtmenge der Wirkstoffe in Gew.-% zur Gesamtmenge der Hilfsstoffe in Gew.-% ins Verhältnis gesetzt wird.

Die erfindungsgemäßen Formkörper dienen der äußeren kosmetischen und pharmazeutischen sowie der oralen oder peroralen Anwendung bei Menschen oder Tieren. Die äußere Anwendung erfolgt dabei so, dass der erfindungsgemäße Formkörper mit Wasser bzw. einer wässrigen Lösung, die gegebenenfalls zusätzlich einen oder mehrere Wirkstoffe und/oder einen oder mehrere Hilfsstoffe enthalten kann, angefeuchtet oder gelöst wird. Je nach Flüssigkeitsmenge und Löslichkeit des Formkörpermaterials das verwendet wird, kann der Formkörper vollständig unter Bildung

einer Lösung gelöst werden oder unter Bildung eines Gels zerfallen um dann anschließend auf das Haar oder die Haut aufgetragen zu werden.

Bevorzugt werden dabei zur Auflösung wässrige Lösungen verwendet, die außerdem mehrwertige Alkohole enthalten können, sowie solche, die niedrigviskos sind (eine Viskosität < 50mPas, aufweisen) und keine oder lediglich geringe Ölanteile (< 10 Gew.-% bezogen auf die Gesamtzusammensetzung der wässrigen Lösung) aufweisen. Weiterhin bevorzugt sind solche Aktivatorlösungen, die frei sind von Erdalkaliionen wie insbesondere Calcium- und/oder Magnesiumionen (weniger als 1 Gew-% Calcium- und/oder Magnesiumionen bezogen auf die Gesamtzusammensetzung der wässrigen Lösung enthalten), sowie solche mit einem pH-Wert zwischen etwa pH 5 bis 7.

Erfindungsgemäß ist in der äußeren Anwendung ebenfalls enthalten, die Lösung des erfindungsgemäßen Formkörpers in einer für eine Badeanwendung geeigneten Wassermenge. Bevorzugt erfolgt die Anwendung jedoch so, dass die Formkörper mit einer kleinen Menge von etwa 0,5 bis 5,0 ml bzw. von ca. dem 10 bis 100fachen des Eigengewichtes des Formkörpers Wasser oder einer Wirk- und/oder Hilfsstofflösung unter Bildung einer Lösung oder eines Gels direkt auf der Haut oder im Haar oder in einem geeigneten Gefäß befeuchtet werden und dort innerhalb von ≤ 30 Sekunden zerfallen und sich vorzugsweise vollständig und rückstandslos auflösen. Bevorzugt erfolgt die Auflösung hierbei ohne mechanische Einwirkung z. B. durch Verrühren, Verreiben, Zerdrücken bzw. Massieren oder lediglich durch derartig leichte mechanische Belastung, die ausreichend ist, um ein Aufbrechen der stabilisierenden Coatinghülle zu bewirken. Bevorzugt ist hierfür bereits die mechanische Belastung ausreichend, die durch Aufbringen einer Aktivatorlösung und dem damit verbundenen hydrostatischen Druck der Aktivatorflüssigkeit hervorgerufen wird.

Die vorliegende Erfindung betrifft auch eine Kombination, enthaltend mindestens einen der erfindungsgemäß verwendeten Formkörper sowie mindestens eine wässrige Lösung, die gegebenenfalls einen oder mehrere Wirkstoffe und/oder einen oder mehrere Hilfsstoffe enthält (eine sogenannte Aktivatorlösung), in einer zusammengehörenden, räumlichen Anordnung (Anwendungspaket, Set, Kit-of-Parts etc.). Bei der Wirkstofflösung kann es sich z.B. um Lösungen von leicht-flüchtigen Wirk- und/oder Hilfsstoffen handeln, die aufgrund des Herstellverfahrens durch die Gefriertrocknung nicht in den Formkörper eingebracht werden sollen bzw. können, wie z.B. gewisse Anteile ätherischer Öle, Parfums etc. Auch können solche Wirk- und/oder Hilfsstoffe enthalten sein, die eine befeuchtende Wirkung erzielen, die insbesondere bei der äußerlichen Anwendung auf der Haut erwünscht und bevorzugt ist, und die aufgrund dieser befeuchtenden Wirkung oder aufgrund von Hygroskopie-Neigungen nicht in den erfindungsgemäßen gefriergetrockneten Formkörper eingearbeitet werden können, da dadurch die Stabilität der feuchtigkeitslabilen Wirkstoffe nicht mehr aufrechterhalten werden kann. Ein Beispiel einer solchen feuchtigkeitsspendenden jedoch hygroskopisch wirkenden Substanz ist beispielsweise Glycerin.

Die Ausgestaltung solcher Kit-of-parts Kombinationen aus erfindungsgemäßem Formkörper einerseits und Wirkstofflösung andererseits kann dabei vorsehen, dass die beiden Bestandteile separat aus der Kit-of-parts Anordnung entnommen werden und außerhalb davon für die weitere Verwendung zusammengeführt und aufgelöst werden. Es ist jedoch auch denkbar, dass eine Zusammenführung der beiden Komponenten innerhalb der Kit-of-parts-Verpackung selbst erfolgt und die aufgelöste Zusammensetzung sodann aus dieser direkt der weiteren kosmetischen oder pharmazeutischen äußerlichen, oralen und/oder peroralen Verwendung zugeführt wird. Dies kann bevorzugt direkt durch den Endverbraucher durchgeführt werden.

Die erfindungsgemäßen Formkörper enthalten ≥ 50 Gew.-%, bevorzugt ≥ 75 Gew.-%, noch bevorzugter ≥ 80 Gew.-%, noch bevorzugter ≥ 90 Gew.% eines oder mehrerer Wirkstoffe, bezogen auf die Gesamtzusammensetzung des gefriergetrockneten, beschichteten Formkörpers. Somit bezieht sich die angegebene Wirkstoffmenge auf einen gefriergetrockneten Formkörper inklusive der Beschichtung mit dem Filmbildner.

Besonders bevorzugt sind feuchtigkeitslabile und/oder saure Wirkstoffe wie insbesondere Ascorbinsäure (Vitamin C) und ihre Derivate und/oder Salicylsäure und ihre Derivate wie Acetylsalicylsäure (ASS).

Der Gehalt der Wirkstoffe in der trockenen Gesamtzusammensetzung kann bestimmt werden durch geeignete anerkannte Analysemethoden wie z. B. nach DIN, Pharm.Eur., Amtliche Sammlung von Untersuchungsverfahren (ASU), DAB, USP etc. Die Wahl der geeigneten Methode hängt dabei natürlich von der Art des Wirkstoffes ab. Insbesondere die besonders bevorzugten Wirkstoffe wie Ascorbinsäure (Vitamin C) und ihre Derivate und/ oder Salicylsäure und ihre Derivate wie Acetylsalicylsäure (ASS) können mittels Hochleistungsflüssigkeitschromatographie-Methoden (HPLC) untersucht werden. HPLC-Methoden zur quantitativen Vitamin C und Acetylsalicylsäurebestimmung können, ggf. mit Anpassungen der Probenaufbereitung, z.B. aus den offiziellen Monografien "Aspirin Tablets" und "Ascorbic acid Injections" aus der USP 31, NF 26 Volume 2, 2008 entnommen werden.

Je nach vorhandener Menge und Art der vorhandenen Wirkstoffe und/oder gegebenenfalls zusätzlichen Hilfsstoffe enthält der erfindungsgemäße Formkörper ≤ 25 Gew.-% eines Gerüstbildners bzw. bevorzugt ≤ 10 Gew.-% eines Gerüstbildners, bezogen auf das Gesamtgewicht des beschichteten gefriergetrockneten Formkörpers, bevorzugter ist ein Anteil von ≤ 7 Gew.-%, noch bevorzugter ≤ 5 Gew.-% des Gerüstbildners, wobei besonders bevorzugt Polysaccharide, wie Natriumalginat oder Chitosan oder auch Carboxymethylcellulose sind.

Der integrale Anteil an Gerüstbildnern in der trockenen Gesamtzusammensetzung kann dabei durch Hydrolyse der vorliegenden Polymerketten mit anschließendem quantitativen, chromatografischem Nachweis der einzelnen Monomerbausteine bestimmt werden. Ist diese Methode durch eine spezielle Kombination unterschiedlicher Gerüstbildner und spezieller Wirk- wie Hilfsstoffe nicht einsetzbar, kann der quantitative Polymeranteil mathematisch über die Differenz zwischen Gesamtgewicht und quantitativ bestimmbaren Hilfs- und Wirkstoffen und Wasser bestimmt werden. Die quantitativen Methoden zur Bestimmung der einzelnen Rezepturkomponenten entlehnt man aus den schon vorher genannten offiziellen Methodensammlungen.

Die Formkörper können bis zu etwa 20 Gew.-% eines oder mehrerer Hilfsstoffe, bevorzugt ≤ 15 Gew.-%, bevorzugter ≤ 10 Gew.-% enthalten.

Bevorzugte Hilfsstoffe sind dabei ausgewählt aus der Gruppe der Fettsubstanzen und Öle, insbesondere aus der Gruppe der kosmetischen Fette und Öle. Ein besonders bevorzugter Hilfsstoff ist Neutralöl (Capryl-/Capronsäure-Triglyceride), Jojobaöl sowie Squalan. Die Formkörper weisen erfindungsgemäß eine Beschichtung oder ein Coating mit mindestens einem Filmbildner auf. Filmbildner schließen ein insbesondere synthetische und natürliche Polymere und Copolymere wie beispielsweise die unter den Hydrokolloiden aufgeführten Strukturpolymeren. Desweiteren können verwendet werden semi-synthetische Cellulosederivate wie z.B. Hydroxypropylmethylcellulose HPMC, Polyvinylacetat (PVA), Polyvinylpyrrolidon (PVP), Shellack, Polyvinylacetatphtalat (PVAP) synthetische Acrylpolymere wie z.B. Methacrylate (Eudragit), Zein etc..

Zusätzlich können noch Polymere eingesetzt werden, welche in Seitz, J.A. Aqueous Film Coating [Encyclopedia of pharmaceutical technology, J.Swarbrick and J.C. Boylan; Marcel Dekker, New York (1988) 1; 337-349] und Cole, G.C. Introduction and overview of pharmaceutical coating, in Pharmaceutical Coating Technology, G. Cole, J. Hogan and M. Aulton; Taylor and Francis LTD., London (1995), 1-5., sowie in Aulton M., Mechanical Properties of film coats, Pharmaceutical coating technology, G. Cole et.al., Taylor and Francis, London (1995), 280-362 genannt sind.

Erfindungsgemäß ist der Filmbildner vorzugsweise ausgewählt aus der Gruppe der hydrophilen Polymere, bevorzugt aus der Gruppe der synthetischen Polymere, besonders bevorzugt aus der Gruppe der Vinylpyrrolidon-Vinylacetat-Copolymere (z.B. Kollidon VA64®).

Der Anteil der Beschichtung mit dem Filmbildner an der gefriergetrockneten Gesamtzusammensetzung inklusive Beschichtung beträgt vorzugsweise ≤ 10 Gew.-%, bevorzugt macht die Beschichtung ≤ 7 Gew.-%, noch bevorzugter ≤ 5 Gew.-% der gecoateten gefriergetrockneten Gesamtzusammensetzung aus.

Die Formkörper enthalten gegebenenfalls auch Reste von Wasser. Da die in den Formkörpern enthaltenen Wirkstoffe jedoch erfindungsgemäß vor insbesondere Feuchtigkeit und außerdem gegen feuchtigkeitsbedingte Instabilität und Auflösung -geschützt werden sollen, ist der Wassergehalt so gering wie möglich zu halten. Je nach Art des Wirkstoffes (hydrophil, hydrophob) kann der Wassergehalt bei bis zu 10 Gew.-% liegen. Der Wassergehalt kann sich nach der Herstellung des Formkörpers durch Gefriertrocknung bei der Lagerung verändern, in der Regel erhöhen. Bevorzugt liegt der Wassergehalt des Formkörpers nach der Herstellung bei maximal 10 Gew.-%, bevorzugt bei weniger als 5 Gew.-%, bevorzugter bei weniger als 1,5 Gew.-%.

Ein besonders bevorzugter Formkörper enthält:

- ≥ 50 Gew.-% eines oder mehrerer Wirkstoffe, insbesondere saure Wirkstoffe wie z. B. Ascorbinsäure (Vitamin C) oder seine Derivate wie Ascorbylglucosid oder Salicylsäure oder seine Derivate wie Acetylsalicylsäure (ASS)
- ≤ 25 Gew.-%, bevorzugter ≤ 10 Gew.-% eines oder mehrerer Gerüstbildner, insbesondere Polysaccharide, wie Natriumalginat, insbesondere calciumfreies Natriumalginat oder kationische Gerüstbildner wie Chitosan und/oder kationisierte Stärke oder modifizierte Gerüstbildner wie Carboxymethylcellulose, insbesondere Natriumcarboxymethylcellulose oder kationisierte Carboxymethylcellulosen sowie Mischungen hieraus
- ≤ 20 Gew.-% eines oder mehrerer Hilfsstoffe, wie insbesondere Fette und Öle, wie z. B. Neutralöl oder Triglyceride,
- ≤ 10 Gew.-% einer Beschichtung mit einem Filmbildner, insbesondere einem synthetischen hydrophilen Polymer wie Vinylpyrrolidon-Vinylacetat-Copolymer (Kollidon VA64®), sowie
- bis zu 10 Gew.-%, bevorzugt bis zu 5 Gew.-%, bevorzugter weniger als 1 Gew.-% Wasser,

mit der Maßgabe, dass der Formkörper bei Flüssigkeitszufuhr in ≤ 30 Sekunden, bevorzugt ≤ 20, bevorzugter ≤ 10, noch bevorzugter ≤ 5 Sekunden vollständig zerfällt und sich auflöst, bevorzugt ohne mechanische Einwirkung.

Bevorzugt weist der erfindungsgemäße Formkörper, wie z. B. der der vorstehend erwähnten Zusammensetzung, enthaltend mindestens 50 Gew.-% eines oder mehrerer Wirkstoffe, ≤ 25 Gew.-% bzw. ≤ 10 Gew.-% eines oder mehrer Gerüstbildner, sowie gegebenenfalls einen oder mehrere Hilfsstoffe, sowie eine Beschichtung mit einem Filmbildner

- eine Dichte von 0,005 g/cm$^3$ bis zu 0,8 g/cm$^3$ bevorzugt 0,01 g/cm$^3$ bis zu 0,8 g/cm$^3$,
- ein Volumen von 0,1 cm$^3$ bis 6 cm$^3$, bevorzugt 0,6 cm$^3$ bis 6 cm$^3$,
- einen Durchmesser (maximaler Abstand zwischen zwei Punkten des Formkörpers) von mindestens 6 mm auf und/oder
- bevorzugt eine sphärische Ausgestaltung, besonders bevorzugt die Form einer Kugel auf.

Die erfindungsgemäßen Formkörper stellen poröse Formkörper mit homogener Verteilung der Inhaltsstoffe im Kern und einer dünnen äußeren Beschichtung mit einem Filmbildner dar.

Dabei können der Beschichtung gegebenenfalls weitere Inhaltsstoffe zugefügt werden wie z. B. Farbstoffe, oder weitere Wirk- und Hilfsstoffe wie z. B. anorganische Salze, Katalysatoren wie beispielsweise Enzyme, Puffersubstanzen, hygroskopisch relevante Substanzen, antimikrobielle Substanzen wie beispielsweise kolloidales Silber oder Silberverbindungen. Insbesondere ist hier denkbar, durch die Beschichtung und die damit einhergehende physikalisch/chemische Inhomogenität des Gesamtformkörpers ein System bereitzustellen, das diese Inhomogenität oder räumliche Trennung in zwei Phasen bewusst ausnutzt, um zwei Phasen voneinander getrennt zu halten, bis die Auflösung und damit vollständige Homogenisierung des gesamten Formkörpermaterials erfolgt. Dies kann z. B. ausgenutzt werden, um eine räumliche Trennung zweier chemischer Reaktanten im inneren Kern und in der äußeren Beschichtung zu erhalten, die eine Reaktion im trockenen, lagerfähigen Zustand verhindert und die erst bei Aktivierung durch Hydratation bzw. Auflösung des Systems zu einer Mobilisierung und damit Reaktion der beiden bisher getrennten immobilisierten Reaktanten führt. Auf diese Weise können beispielsweise stabilere Wirkstoffderivate im Kern der Zusammensetzung bereitgestellt werden und die chemischen Umsetzungsagentien, die aus diesen Derivaten oder Precursorn die aktiven aber instabileren Wirkstoffe freisetzen, in der äußeren Umhüllung. Durch die räumliche Trennung und Immobilisierung in getrennten Schichten wird eine vorzeitige Reaktion verhindert. Auch ist es möglich, durch Zusatz von Farbstoffen in der Umhüllungsschicht farblich ansprechende Formkörper zur Verfügung zu stellen. Durch die Konzentration der Farbstoffe z.B. in einer äußeren dünnen Coatingschicht ist mit wenig Farbstoff eine deutlich stärkere Farbgebung zu erreichen, als wenn die gleiche Farbstoffmenge homogen in der gesamten Formkörper-Zusammensetzung verteilt vorliegt. Dies ist vorteilhaft, um mit geringen Farbstoffgehalten eine gute Farbgebung zu erzielen, ohne dass durch zu hohe Farbstoffanteile bei der äußerlichen Anwendung unerwünschte färbende Rückstände auf der Haut verbleiben.

Die erfindungsgemäßen Formkörper wie beispielsweise solche wie vorstehend genannt, werden bevorzugt mit einer wässrigen Flüssigkeit / Aktivatorlösung aufgelöst, die enthält:

- mindestens 70 Gew.-% Wasser,
- mindestens 5 Gew.-% mehrwertige Alkohole,
- bis zu 10 Gew.-% eines oder mehrerer Wirkstoffe wie insbesondere solche aus der Gruppe der kosmetischen Wirkstoffe
- bis zu 20 Gew.-% eines oder mehrerer Hilfsstoffe, wie insbesondere solche aus der Gruppe der kosmetischen Öle, wie insbesondere Capryl/Capronsäure-Triglyceride oder Jojobaöl und die einen pH-Wert von 5 - 7 und des weiteren einen Gehalt an Erdalkaliionen wie insbesondere Calcium- und/oder Magnesiumionen von weniger als 1 Gew-% aufweist.

Die Auflösungsgeschwindigkeit der erfindungsgemäßen Formkörper, gemessen entsprechend einer Methode zur Messung der "Zerfallszeit von Tabletten und Kapseln" mit einer Testapparatur nach PharmEU beträgt weniger als 30 Sekunden, bevorzugt weniger als 20 Sekunden, bevorzugter weniger als 10 Sekunden, besonders bevorzugt weniger als 5 Sekunden.

Gegenstand der Erfindung ist außerdem ein Verfahren, zur Herstellung eines gefriergetrockneten beschichteten Formkörpers das dadurch gekennzeichnet ist, dass die Beschichtung auf dem gefrorenen Formkörper erfolgt und der beschichtete gefrorene Formkörper anschließend gefriergetrocknet wird.
Insbesondere ist Gegenstand der Erfindung ein Verfahren,
das die folgenden Schritte umfasst:

(a) Herstellen einer wässrigen Lösung oder Suspension eines oder mehrerer Wirkstoffe, gegebenenfalls eines oder mehrerer Gerüstbildner, sowie gegebenenfalls eines oder mehrerer Hilfsstoffe,
(b) Giessen der Mischung in eine Form
(c) Gefrieren der Mischung in der Form unter Erhalt von gefrorenen Formkörpern
(d) Entnehmen der gefrorenen Formkörper aus der Form und gegebenenfalls Rondieren der gefrorenen Formkörper
(e) Besprühen der gefrorenen Formkörper mit einer Beschichtungszusammensetzung, enthaltend mindestens einen Filmbildner sowie mindestens ein Lösungs- und/oder Dispergiermittel
(f) Gefriertrocknung der gefrorenen und mit dem Filmbildner beschichteten gefrorenen Formkörper unter Bildung des gefriergetrockneten Formkörpers.

Zweckmäßig geht man bei der Herstellung so vor, dass man zunächst eine wässrige Lösung der Wirkstoffe herstellt und gegebenenfalls eine Lösung des Gerüstbildners unter Rühren einmischt. Anschließend gibt man der Mischung gegebenenfalls weitere Hilfsstoffe hinzu und vermischt.

Die Menge der in der Lösung bzw. Suspension enthaltenen Feststoffe wie Gerüstbildner, Wirkstoffe und Hilfsstoffe beeinflusst maßgeblich die Dichte (Gewicht des Formkörpers bezogen auf das Volumen der geometrischen Form

des Formkörpers) des erhaltenen Formkörpers. Die Dichte ist wiederum eine wichtige Größe für die Porosität des Formkörpers und damit wiederum für die Auflösungsgeschwindigkeit des Formkörpers beim Befeuchten mit Wasser oder einer Wirk- und/oder Hilfsstofflösung. Die poröse Struktur gefriergetrockneter Formkörper ist eine wesentliche Grundlage für die schnelle Löslichkeit, da durch die große Oberfläche im porösen Material ein inniger Austausch zwischen wässriger Phase und festem Formkörper während des Rehydratisierungsprozesses stattfinden kann. Je höher die Konzentration der Wirkstoffe, des Gerüstbildners sowie ggf. der Hilfsstoffe in der Lösung ist, umso höher wird die Dichte und damit umso geringer wird der Porositätsgrad des Formkörpers und umgekehrt. Allerdings hängt der Porositätsgrad der Formkörper nicht allein von der Materialdichte ab. Vielmehr ist die Materialporosität im wesentlichen eine Funktion zweier Parameter, der Materialdichte und der Eiskristallgröße. Hohe Feststoffgehalte in der wässrigen Suspension erhöhen die Materialdichte im gefriergetrockneten Endprodukt und verringern die Kontaktfläche Rehydratisierungsmittel/Feststoff. Hohe Einfriergradienten führen zu kleinen Eiskristallen, welche zu großen inneren Materialoberflächen führen, was wiederum die Rehydratisierung begünstigt. Zur schnellen Befeuchtung und Auflösung der gefriergetrockneten Formkörper sind also niedrige Materialdichten und kleine Eiskristalle vorteilhaft.

Da durch die Beschichtung der gefrorenen Formkörper inhomogene Materialien erhalten werden und der im inneren der Formkörper erhältliche poröse Kern nach außen hin durch eine filmbildende Schicht abgeschirmt ist, ist für die Auflösung der beschichteten Formkörper außerdem die Beschaffenheit der Filmbeschichtung und ihr Verhalten gegenüber den zur Auflösung verwendeten Medien von elementarer Bedeutung. Insbesondere poröse Filmbeschichtungen ermöglichen einen Transport der Flüssigkeiten in den hochporösen Kern und somit eine schnelle Auflösung der Formkörper. Da insbesondere für die kosmetische und pharmazeutische Anwendung bevorzugt Auflösungsmedien auf wässriger Basis verwendet werden, ist es wichtig, die Beschichtung aus der Gruppe der hydrophilen Filmbildner auszuwählen um Probleme bei der Befeuchtung und Auflösung der beschichteten Formkörper zu vermeiden. Weiterhin ist die Feuchtigkeitsdurchlässigkeit des Filmbildners beim Gefriertrocknungsprozess aus vorstehend aufgeführten Gründen relevant, da bei der Sublimation der Wasserdampf durch die Filmschicht aus dem Formkörper entweichen muss.

Unter dem Gesichtspunkt Dichte/Porositätsgrad bzw. der Auflösungsgeschwindigkeit wird die Rezepturierung und Herstellung der erfindungsgemäßen Formkörper so ausgerichtet, dass die Dichten der damit erhältlichen Formkörper zweckmäßig bei etwa 0,01 g/cm$^3$ bis zu 0,8 g/cm$^3$, bevorzugt etwa 0,015 g/cm$^3$ bis zu 0,5 g/cm$^3$, bevorzugt etwa 0,02 g/cm$^3$ bis zu 0,3 g/cm$^3$ liegen. Der Begriff der Dichte, wie er vorliegend verwendet wird, bezeichnet das Gewicht des Formkörpers bezogen auf das Volumen der äußeren geometrischen Form des Formkörpers.

Das Gewicht der einzelnen Formkörper hängt natürlich von deren Größe ab. Im allgemeinen liegt das Gewicht der einzelnen Formkörper bei etwa 10 bis 300 mg, bevorzugt 20 bis 200 mg. Beispielsweise weisen Kugeln von 11 mm Durchmesser ein Gewicht im Bereich von bevorzugt 20 bis 160 mg, bevorzugter 30 bis 150 mg auf. Für Kugeln anderer Durchmesser berechnen sich entsprechend der Volumenänderung andere Vorzugsbereiche.

Die Herstellung der Lösung, die der Gefriertrocknung unterworfen wird, erfolgt bevorzugt so, dass zunächst eine wässrige Lösung der Wirkstoffe hergestellt wird, in die gegebenenfalls eine Lösung eines oder mehrerer Gerüstbildner eingemischt wird. Anschließend werden der Mischung gegebenenfalls weitere Hilfsstoffe zugemischt. Werden öllösliche Wirkstoffe verwendet, werden diese bevorzugt in gegebenenfalls als Hilfsstoffe verwendeten Ölen (insbesondere Squalan und Triglyceriden) gelöst und anschließend der wässrigen Lösung der Wirkstoffe oder des Gerüstbildners zugesetzt. Diese Herstellweise besitzt den Vorteil, dass sich kurzzeitig stabile Lösungen bzw. Suspensionen bilden. Es werden keine Emulgatoren oder nur geringe Mengen oberflächenaktiver Substanzen wie z. B. Tenside oder Netzmittel benötigt, und es findet während der Verarbeitung keine Phasentrennung der Lösung oder Suspension bei Verwendung öllöslicher bzw. öliger Hilfs- bzw. Wirkstoffe statt. Bevorzugt werden jedoch wasserlösliche Wirkstoffe verwendet.

Die so hergestellte Lösung wird dann in Formen gegossen, die den Formkörpern entsprechende Hohlräume der gewünschten geometrischen Formen aufweisen. Die Form besteht bevorzugt aus Kautschuk, Silikon-Kautschuk, vulkanisiertem Kautschuk (Gummi) etc. Bevorzugt sind Gummiformen. Die Formmaterialien können gegebenenfalls beschichtet sein. Die Hohlräume der Formkörper, in die die Lösung hineingegossen wird, weisen im allgemeinen die Form des gewünschten Formkörpers auf. D.h., dass das Volumen des Hohlraums im wesentlichen dem Volumen der später erhaltenen Formkörper entspricht.

Da das Volumen der in die Hohlräume eingefüllten Lösungen bzw. Suspensionen beim Gefrieren zunimmt (Dichteunterschied zwischen Wasser und Eis), werden die Hohlräume in der Regel nicht vollständig gefüllt. Es werden auf diese Weise vollständig symmetrische Formkörper erhalten. Dies ist beispielsweise nach dem Verfahren durch Eintropfen in tiefkalte Lösungen (wie in flüssigen Stickstoff) nicht möglich, da es dort zu unsymmetrischen Temperaturverteilungen kommt, so dass sich stets mehr oder weniger starke Abweichungen von einer regelmäßigen Form ergeben. Solche unregelmäßig geformten Formkörper sind jedoch gerade im Bereich der kosmetischen Endprodukte nicht erwünscht. Das bedeutet in der Regel, dass diese nach dem Eintropfverfahren hergestellten Formkörper einer mechanischen Nachbearbeitung bedürfen, was nach dem Verfahren, wie es erfindungsgemäß verwendet wird, nicht erforderlich ist. Bei nach dem Eintropfverfahren hergestellten Formkörpern wird eine solche Nachbearbeitung mit zunehmendem Volumen des Formkörpers immer erforderlicher, da bei diesem Verfahren deutliche äußerliche Unregelmäßigkeiten auftreten, die insbesondere bei größeren Formkörpern stärker in Erscheinung treten.

Nach dem Einfüllen der Lösung in die Hohlräume der Form wird die Lösung bzw. Suspension gefroren. Das Abkühlen bzw. Gefrieren der Lösung kann an sich in beliebiger Weise erfolgen, wie beispielsweise durch Anblasen mit kalter Luft, Abkühlen durch Aufbringen auf eine mit Kühlsole durchflossene Platte oder auch das Eintauchen der Formen in flüssige Gase, wie z.B. das Eintauchen in flüssigen Stickstoff. Die Abkühlgeschwindigkeit beeinflusst dabei die Größe der gebildeten Eiskristalle. Diese beeinflussen wiederum die Porengrößenverteilung im inneren Kern des gebildeten Formkörpers. Werden wenige große Kristalle gebildet, so weist der Formkörper wenige große Poren auf, werden viele kleine Kristalle gebildet, weist der Formkörper viele kleine Poren auf. Die Kristalle werden umso kleiner, je höher die Abkühlgeschwindigkeit der Lösung bzw. Suspension ist. Dabei wird eine Einfriergeometrie bevorzugt, bei der die Formkörper bei mindestens < -20 °C in der Form gleichzeitig von allen Seiten eingefroren werden.

Die Gefriertemperatur, die erforderlich ist, hängt unter anderem davon ab, wie stark die Gefrierpunktserniedrigung durch die in der Lösung enthaltenden Wirkstoffe bzw. Hilfsstoffe ist. Zweckmäßig liegt die Temperatur unterhalb des Gefrierpunktes von Wasser bis zur Temperatur von flüssigem Stickstoff (- 196°C). Bevorzugt ist die Gefriertemperatur etwa -20 bis -80°C, besonders bevorzugt -30 bis - 50 °C Nach dem Gefrieren der Lösung bzw. Suspension werden die Formkörper aus der Form genommen und gegebenenfalls rondiert. Danach wird das Coating durch Besprühen der gefrorenen Formkörper mit einer geeigneten Beschichtungszusammensetzung, enthaltend mindestens einen Filmbildner sowie mindestens ein Lösungs- und/oder Dispergiermittel aufgebracht. Vorzugsweise handelt es sich bei einem solchen Lösungs- und/oder Dispergiermittel um ein Wasser-Alkohol-Gemisch.

Bei der Beschichtungszusammensetzung handelt es sich bevorzugt um eine Zusammensetzung von ≥ 15 Gew.-% Alkohol, ≤ 70 Gew.-% Wasser und ≥ 5 Gew.-% Filmbilder, jeweils bezogen auf die Gesamtmenge der Beschichtungszusammensetzung.

Gegebenenfalls kann die wässrige Lösung des hydrophilen Filmbildners noch gefrierpunktserniedrigende Substanzen wie z.B. ein- oder mehrwertige Alkohole oder Salze enthalten. Durch die gefrierpunktserniedrigenden Substanzen wird eine vorzeitige Eisbildung beim Sprühprozess vermieden. Zudem wir durch diese Zusätze die Außenseite des gefrorenen Formkörpers kurz angetaut und das filmbildende Polymer kann einen stabilen, mit dem gefrorenen Formkörper fest verbundenen Film ausbilden. Bevorzugt werden gefrierpunktserniedrigende Substanzen gewählt, welche im Rahmen der Gefriertrocknung wieder vom Formkörper entfernt werden können. Dazu zählen besonders die einwertigen Alkohole Methanol und Ethanol. Es ist auch möglich, die gefrorenen Formkörper in die Lösung des Filmbildners zu rollen oder zu tauchen. Dabei weist die Lösung des Filmbildners in dem Lösungs- und/oder Dispergiermittel, vorzugsweise in einem Wasser-Alkohol-Gemisch vorzugsweise eine Zusammensetzung aus mindestens 5 Gew.-% Alkohol, mindestens 50 Gew.-% Wasser und mindestens 10 Gew.-% Filmbildner, jeweils bezogen auf die Gesamtmenge der Lösung, auf. Prinzipiell ist es möglich, den Filmbildner in allen mit Wasser mischbaren Alkoholen oder Lösungsmitteln zu lösen. Besonders bevorzugt wird jedoch ein Wasser-Ethanol-Gemisch verwendet.

Gegebenenfalls können dem Gemisch aus Wasser, Alkohol und Filmbildner weitere darin lösliche Substanzen wie Farb-, Wirk- oder Hilfsstoffe zugesetzt werden, sowie geeignete Substanzen, zur chemischen Umsetzung von Wirkstoffderivaten und/oder -precursorn.

Anschließend werden die derart beschichteten Formkörper der Gefriertrocknung unterworfen. Die Gefriertrocknung kann in an sich bekannter Weise mittels allgemein bekannter Gefriertrocknungsverfahren, wie z.B. auch in der DE 4328329 C2, in der DE 4028622 C2 oder in der DE 10350654 A1 beschrieben, erfolgen.

Die Erfindung umfasst insbesondere die folgenden bevorzugten Ausführungsformen:

1. Gefriergetrockneter Formkörper enthaltend: einen oder mehrere Wirkstoffe und gegebenenfalls einen oder mehrere Gerüstbildner, gegebenenfalls einen oder mehrere Hilfsstoffe, sowie eine Oberflächen-Beschichtung, enthaltend mindestens einen Filmbildner.

2. Gefriergetrockneter Formkörper nach Ausführungsform 1 mit einem Wirkstoffanteil ≥ 50 Gew. %, bevorzugt ≥ 75 Gew. %, bevorzugter ≥ 80 Gew. %, noch bevorzugter ≥ 90 Gew. % bezogen auf die Gesamtzusammensetzung des gefriergetrockneten beschichteten Formkörpers.

3. Gefriergetrockneter Formkörper nach einer der Ausführungsformen 1 oder 2 mit einem Gesamtanteil an Wirk- und Hilfsstoffen von ≥ 50 Gew. %, bevorzugt ≥ 75 Gew. %, bevorzugter ≥ 80 Gew. %, noch bevorzugter ≥ 90 Gew. % bezogen auf die Gesamtzusammensetzung des gefriergetrockneten beschichteten Formkörpers.

4. Gefriergetrockneter Formkörper nach einer der Ausführungsformen 1 bis 3, mit einem Gewichtsverhältnis von Wirkstoffen zu Hilfsstoffen von 10:1 bis 100:1 jeweils bezogen auf die Gesamtmenge der Wirkstoffe in Gew.-% im Verhältnis zur Gesamtmenge der Hilfsstoffe in Gew.-%

5. Gefriergetrockneter Formkörper nach einer der Ausführungsformen 1 bis 4, worin eine 1 gewichtsprozentige Lösung oder Suspension des Wirkstoffs in Wasser bei 20 °C einen pH Wert < 7 aufweist.

6. Gefriergetrockneter Formkörper nach einer der Ausführungsformen 1 bis 4, worin der Wirkstoff ausgewählt ist aus der Gruppe der sauren Wirkstoffe mit einen pKs-Wert bei 25 °C von ≤ 7.

7. Gefriergetrockneter Formkörper nach einer der Ausführungsformen 1 bis 6, worin der Wirkstoff ausgewählt ist aus der Gruppe, die besteht aus Ascorbinsäure und deren Derivate, Salicylsäure und deren Derivate, insbesondere Acetylsalicylsäure, Clofibrinsäure, Ibuprofen, Gemfibrozil, Fenoprofen, Naproxen, Ketoprofen, Indomethacin, Bezafibrat, Tolfenaminsäure, Diclofenac, Moclofenaminsäure, Paracetamol, Acitretin, Acrivastin, Azelainsäure, Cromolyn, Ethacrynsäure, Furosemid, Penicillin und Derivate davon, Vitamin A und Derivate davon, Risedronsäure und Derivate davon, Liponsäure, und Ursodiol.

8. Gefriergetrockneter Formkörper nach Ausführungsform 7, worin mindestens ein saurer Wirkstoff ausgewählt ist aus der Gruppe der Ascorbinsäure und deren Derivate oder aus der Gruppe der Acetylsalicylsäure und deren Derivate.

9. Gefriergetrockneter Formkörper nach einer der Ausführungsformen 1 bis 8, der bezogen auf die Gesamtzusammensetzung des gefriergetrockneten beschichteten Formkörpers ≥ 50 Gew. % eines Wirkstoffs aus der Gruppe der Ascorbinsäure und ihrer Derivate enthält.

10. Gefriergetrockneter Formkörper nach einer der Ausführungsformen 1 bis 8, der bezogen auf die Gesamtzusammensetzung des gefriergetrockneten beschichteten Formkörpers ≥ 50 Gew.% eines Wirkstoffs aus der Gruppe der Salicylsäure und deren Derivate, bevorzugt aus der Gruppe der Acetylsalicylsäure und ihrer Derivate enthält.

11. Gefriergetrockneter Formkörper einer der Ausführungsformen 1 bis 10 mit einem Gerüstbildneranteil ≤ 25 Gew. %, bevorzugt ≤ 10 Gew. %, bevorzugter ≤ 5 Gew. %, bezogen auf die Gesamtzusammensetzung des gefriergetrockneten beschichteten Formkörpers.

12. Gefriergetrockneter Formkörper nach einer der Ausführungsformen 1 bis 11 worin keine Gerüstbildner enthalten sind.

13. Gefriergetrockneter Formkörper nach einer der Ausführungsformen 1 bis 12, worin der Gerüstbildner ausgewählt ist aus der Gruppe der Hydrokolloide, bevorzugt aus der Gruppe der Polysaccharide, Polyaminosaccharide, Glucosaminoglycane und/oder synthetischen Polymere oder Mischungen davon.

14. Gefriergetrockneter Formkörper nach Ausführungsform 13, worin der Gerüstbildner ein Alginat, bevorzugt ein Natrium-Alginat, Carboxymethylcellulose, kationisch modifizierte Carboxymethylcellulose, kationisch modifizierte Stärke oder Chitosan oder eine Mischung davon ist.

15. Gefriergetrockneter Formkörper nach einer der Ausführungsformen 1 bis 14, worin Gerüstbildner aus der Gruppe der Proteine ausgenommen sind.

16. Gefriergetrockneter Formkörper nach einer der Ausführungsformen 1 bis 15, worin der Anteil der Beschichtung mit einem Filmbildner ≤ 10 Gew.-%, bevorzugt ≤ 7 Gew.-%, bevorzugter ≤ 5 Gew.-%, jeweils bezogen auf die Gesamtzusammensetzung des gefriergetrockneten beschichteten Formkörpers, beträgt.

17. Gefriergetrockneter Formkörper nach einer der Ausführungsformen 1 bis 16 worin der Filmbildner für die Beschichtung ausgewählt ist aus der Gruppe der hydrophilen Polymere.

18. Gefriergetrockneter Formkörper nach Ausführungsform 17 worin der Filmbildner für die Beschichtung ausgewählt ist aus der Gruppe der synthetischen Polymere, bevorzugt aus der Gruppe der Vinylpyrrolidon-Vinylacetat-Copolymere.

19. Gefriergetrockneter Formkörper einer der Ausführungsformen 1 bis 18, **dadurch gekennzeichnet, daß** er bei Flüssigkeitszufuhr in ≤ 30 Sekunden, bevorzugt ≤ 20 Sekunden, bevorzugter ≤ 10 Sekunden, noch bevorzugter ≤ 5 Sekunden vollständig zerfällt.

20. Gefriergetrockneter Formkörper nach einer der Ausführungsformen 1 bis 19 der ein Volumen von 0,1 $cm^3$ bis 6 $cm^3$, eine Dichte von 0,01 $g/cm^3$ bis 0,8 $g/cm^3$ und/oder die geometrische Form einer Kugel mit einem Durchmesser von mindestens 6 mm aufweist.

21. Verfahren zur Herstellung eines gefriergetrockneten beschichteten Formkörpers das dadurch gekennzeichnet ist, daß die Beschichtung auf dem gefrorenen Formkörper erfolgt und der beschichtete gefrorene Formkörper anschließend gefriergetrocknet wird.

22. Verfahren zur Herstellung eines gefriergetrockneten Formkörpers das die Schritte umfasst

a. Herstellen einer wässrigen Lösung oder Suspension eines oder mehrerer Wirkstoffe, gegebenenfalls eines oder mehrerer Gerüstbildner sowie gegebenenfalls eines oder mehrerer Hilfsstoffe,
b. Giessen der Mischung in eine Form
c. Gefrieren der Mischung in der Form unter Erhalt von gefrorenen Formkörpern
d. Entnehmen der gefrorenen Formkörper aus der Form und gegebenenfalls Rondieren
e. Besprühen der gefrorenen Formkörper mit einer Beschichtungszusammensetzung, enthaltend mindestens einen Filmbildner sowie mindestens ein Lösungs- und/oder Dispergiermittel
f. Gefriertrocknung der mit dem Filmbildner beschichteten gefrorenen Formkörper unter Bildung des gefriergetrockneten Formkörpers.

23. Verfahren nach einer der Ausführungsformen 21 oder 22, worin zur Beschichtung ein Filmbildner verwendet wird, der ausgewählt ist aus der Gruppe der hydrophilen Polymere, bevorzugt aus der Gruppe der synthetischen Polymere, besonders bevorzugt aus der Gruppe der Vinylpyrrolidon-Vinylacetat-Copolymere.

24. Verfahren nach Ausführungsform 22 oder 23, worin das Lösungs- und/oder Dispergiermittel ein Wasser-Alkohol-Gemisch ist.

25. Verfahren nach einer der Ausführungsformen 22 bis 24, worin die Beschichtungszusammensetzung eine Zusammensetzung von ≥ 15 Gew.-% Alkohol, ≤ 70 Gew.-% Wasser und ≥ 5 Gew.-% Filmbilder, jeweils bezogen auf die Gesamtmenge der Beschichtungszusammensetzung ist.

26. Gefriergetrockneter Formkörper erhältlich nach dem Verfahren nach einer der Ausführungsformen 21 bis 25.

27. Verwendung des gefriergetrockneten Formkörpers nach einer der Ausführungsformen 1 bis 20 oder 26 als kosmetisches Mittel.

28. Verwendung des gefriergetrockneten Formkörpers nach einer der Ausführungsformen 1 bis 20 oder 26 als pharmazeutisches Mittel.

29. Verwendung nach Ausführungsform 27 oder 28, worin die Anwendung äußerlich erfolgt.

30. Verwendung nach einer der Ausführungsformen 27 bis 29, worin der gefriergetrocknete Formkörper mit Wasser oder einer wässrigen Lösung eines oder mehrerer Wirkstoffe und/oder gegebenenfalls Hilfsstoffe angefeuchtet wird und in ≤ 30 Sekunden zerfällt und anschließend auf die Haut oder auf das Haar aufgetragen wird

31. Verwendung des gefriergetrockneten Formkörpers nach einer der Ausführungsformen 1 bis 20 oder 26 zur oralen oder peroralen Applikation von Wirkstoffen.

32. Kit-of-parts Kombination enthaltend mindestens einen gefriergetrockneten Formkörper gemäß einer der Ausführungsformen 1 bis 20 oder 26 sowie mindestens eine wässrige Lösung, die einen oder mehrere Wirkstoffe und/oder gegebenenfalls einen oder mehrere Hilfsstoffe enthält, in zusammengehöriger, räumlicher Anordnung.

33. Verwendung der Kit-of-parts Kombination nach Ausführungsform 32 als kosmetisches Mittel.

34. Verwendung der Kit-of-parts Kombination nach Ausführungsform 32 als therapeutisches Mittel.

35. Verwendung nach einer der Ausführungsformen 27 bis 31 und 33 bis 34, die direkt durch den Endverbraucher erfolgt.

Die Erfindung wird durch die folgenden Beispiele näher veranschaulicht.

BEISPIELE

**Beispiel 1**

**Herstellung einer Carboxymethylcellulose / Ascorbinsäure-Kugel mit anschließendem Beschichten**

| | |
|---|---|
| 0,5 g | Carboxymethylcellulose |
| 16,0 g | Ascorbinsäure |
| 83,5 g | Wasser |

0,5 g Carboxymethylcellulose werden unter Rühren in 83,5 g Wasser gegeben und bis zur vollständigen, homogenen Auflösung der Carboxymethylcellulose gerührt. Anschließend wird unter Rühren 16,0 g Ascorbinsäure hinzugegeben, dabei wird die Mischung, die einen pH-Wert ≤ 3,0 aufweist, auf einer Temperatur von 0-10 °C gehalten. Die homogene (entgaste) Mischung wird in Formen gegossen, unter Anblasen von kalter Luft ausgefroren und aus der Form genommen und gegebenenfalls mechanisch nachbearbeitet. Man erhält gefrorene Kugeln mit einem Durchmesser von ca. 11 mm, welche gegebenenfalls im gefrorenen Zustand bei Temperaturen unter -20°C gelagert werden können. Die gefrorenen Formkörper werden in einem Kühlraum bei Temperaturen < -10°C unter Schütteln homogen mit einer Lösung aus: 80g Ethanol
10g RO-Wasser (reverse osmosis Wasser)
10g Kollidon VA-64
besprüht, bis alle Kugeln gleichmäßig mit dem Überzugsmittel überzogen sind. Unter Schütteln lässt man das überschüssige Ethanol abdampfen. Die überzogenen, tiefgekühlten Kugeln werden anschließend einer Gefriertrocknung unterworfen. Man erhält mit Kollidon VA-64 überzogene, mechanisch stabile gefriergetrocknete Formkörper.
Die Menge an Überzugsmittel und damit die mechanische Stabilität des gefriergetrockneten Formkörpers, kann leicht über die Menge an aufgesprühtem Überzugsmittel gesteuert werden.
Die Auflösegeschwindigkeit der gefriergetrockneten, beschichteten Formkörper, gemessen nach einer Methode zur Messung der "Zerfallszeit von Tabletten und Kapseln" mit einer Testapparatur nach PharmEU, beträgt weniger als 10 Sekunden.

**Beispiel 2**

**Herstellung einer Chitosan/Acetylsalicylsäure-Kugel mit anschließendem Beschichten**

| | |
|---|---|
| 0,2 g | Chitosan |
| 16,0 g | Acetylsalicylsäure |
| 83,8 g | Wasser |

0,2 g Chitosan wird unter Rühren in 83,8 g Wasser gegeben, mit etwas verdünnter Salzsäure versetzt und bis zur vollständigen Auflösung des Chitosans gerührt. Anschließend werden unter Rühren 16,0 g Acetylsalicylsäure in die Chitosanlösung dispergiert, dabei wird die Mischung, die einen pH-Wert ≤ 3,0 aufweist, auf einer Temperatur von 0-10 °C gehalten. Die homogene (entgaste) Mischung wird in Formen gegossen, unter Anblasen von kalter Luft ausgefroren und aus der Form genommen und gegebenenfalls mechanisch nachbearbeitet. Man erhält gefrorene Kugeln mit einem Durchmesser von ca. 11 mm, welche gegebenenfalls im gefrorenen Zustand bei Temperaturen unter -20°C gelagert werden können.
Die gefrorenen Formkörper werden in einem Kühlraum bei Temperaturen < -10°C unter Schütteln und Anblasen mit kalter Luft homogen mit einer Lösung aus:

| | |
|---|---|
| 85g | Ethanol |
| 15g | Kollidon VA-64 |

besprüht, bis alle Kugeln gleichmäßig mit dem Überzugsmittel überzogen sind. Unter Schütteln und anblasen mit kalter Luft lässt man das überschüssige Ethanol abdampfen. Die überzogenen, tiefgekühlten Kugeln werden anschließend

einer Gefriertrocknung unterworfen. Man erhält mit Kollidon VA-64 überzogene, mechanisch stabile gefriergetrocknete Formkörper.
Die Menge an Überzugsmittel und damit die mechanische Stabilität des gefriergetrockneten Formkörpers, kann leicht über die Menge an aufgesprühtem Überzugsmittel gesteuert werden.

Die Auflösegeschwindigkeit der gefriergetrockneten, beschichteten Formkörper, gemessen nach einer Methode zur Messung der "Zerfallszeit von Tabletten und Kapseln" mit einer Testapparatur nach PharmEU, beträgt weniger als 10 Sekunden.

**Beispiel 3**

**Herstellung einer Ascorbinsäure-Kugel mit anschließendem Beschichten**

| | |
|---|---|
| 16,0 g | Ascorbinsäure |
| 84,0 g | Wasser |

16,0 g Ascorbinsäure werden in 84g Wasser aufgelöst. Der pH-Wert der Lösung ist ≤ pH 3,0 und die Lösung wird auf einer Temperatur von 0-10 °C gehalten. Die homogene (entgaste) Lösung wird in Formen gegossen, unter Anblasen von kalter Luft ausgefroren, aus der Form genommen und gegebenenfalls mechanisch nachbearbeitet. Man erhält gefrorene Ascorbinsäure-Kugeln mit einem Durchmesser von ca. 11 mm, welche gegebenenfalls im gefrorenen Zustand bei Temperaturen unter -20°C gelagert werden können.
Die gefrorenen Formkörper werden in einem Kühlraum bei Temperaturen < -10°C unter Schütteln homogen mit einer Lösung aus:

| | |
|---|---|
| 90g | Ethanol |
| 10g | Kollidon VA-64 |

besprüht, bis alle Kugeln gleichmäßig mit dem Überzugsmittel überzogen sind. Unter Schütteln lässt man das überschüssige Ethanol abdampfen. Die überzogenen, tiefgekühlten Kugeln werden anschließend einer Gefriertrocknung unterworfen. Man erhält mit Kollidon VA-64 überzogene, mechanisch stabile gefriergetrocknete Formkörper aus Ascorbinsäure.

Die Menge an Überzugsmittel und damit die mechanische Stabilität des gefriergetrockneten Formkörpers, kann leicht über die Menge an aufgesprühtem Überzugsmittel gesteuert werden.

Die Auflösegeschwindigkeit der gefriergetrockneten, beschichteten Formkörper, gemessen nach einer Methode zur Messung der "Zerfallszeit von Tabletten und Kapseln" mit einer Testapparatur nach PharmEU, beträgt weniger als 5 Sekunden.

**Beispiel 4**

**Herstellung einer kationisierte Stärke / Ascorbinsäure-Kugel mit anschließendem Beschichten**

| | |
|---|---|
| 2,0 g | kationisierte Stärke |
| 1,0 g | Jojobaöl |
| 16,0 g | Ascorbinsäure |
| 81 ,0 g | Wasser |

2,0 g kationisierte Stärke wird unter Rühren in 81,0 g Wasser gegeben und bis zur vollständigen, homogenen Auflösung der Stärke gerührt. Anschließend wird unter Rühren 16,0 g Ascorbinsäure und 1 g Jojobaöl hinzugegeben, dabei wird die Mischung, die einen pH-Wert ≤ 3,0 aufweist, auf einer Temperatur von 0-10 °C gehalten. Die homogene (entgaste) Mischung wird in Formen gegossen, unter Anblasen von kalter Luft ausgefroren und aus der Form genommen und gegebenenfalls mechanisch nachbearbeitet. Man erhält gefrorene Kugeln mit einem Durchmesser von ca. 11 mm, welche gegebenenfalls im gefrorenen Zustand bei Temperaturen unter -20°C gelagert werden können.
Die gefrorenen Formkörper werden in einem Kühlraum bei Temperaturen < -10°C unter Schütteln homogen mit einer

Lösung aus:

| | |
|---|---|
| 80g | Ethanol |
| 10g | RO-Wasser |
| 10g | Kollidon VA-64 |

besprüht, bis alle Kugeln gleichmäßig mit dem Überzugsmittel überzogen sind. Unter Schütteln lässt man das überschüssige Ethanol abdampfen. Die überzogenen, tiefgekühlten Kugeln werden anschließend einer Gefriertrocknung unterworfen. Man erhält mit Kollidon VA-64 überzogene, mechanisch stabile gefriergetrocknete Formkörper.

Die Menge an Überzugsmittel und damit die mechanische Stabilität des gefriergetrockneten Formkörpers, kann leicht über die Menge an aufgesprühtem Überzugsmittel gesteuert werden.

Die Auflösegeschwindigkeit der gefriergetrockneten, beschichteten Formkörper, gemessen nach einer Methode zur Messung der "Zerfallszeit von Tabletten und Kapseln" mit einer Testapparatur nach PharmEU, beträgt weniger als 10 Sekunden.

**Patentansprüche**

1. Gefriergetrockneter Formkörper enthaltend einen oder mehrere Wirkstoffe und gegebenenfalls einen oder mehrere Gerüstbildner, gegebenenfalls einen oder mehrere Hilfsstoffe, sowie eine Beschichtung, enthaltend mindestens einen Filmbildner.

2. Gefriergetrockneter Formkörper nach Anspruch 1 mit einem Wirkstoffanteil ≥ 50 Gew. %, bevorzugt ≥ 75 Gew. %, bevorzugter ≥ 80 Gew. %, noch bevorzugter ≥ 90 Gew. % bezogen auf die Gesamtzusammensetzung des gefriergetrockneten beschichteten Formkörpers.

3. Gefriergetrockneter Formkörper nach einem der Ansprüche 1 oder 2, worin der Wirkstoff ausgewählt ist aus der Gruppe der sauren Wirkstoffe mit einen pKs-Wert bei 25 °C von ≤ 7.

4. Gefriergetrockneter Formkörper nach einem der Ansprüche 1 bis 3 der bezogen auf die Gesamtzusammensetzung des gefriergetrockneten beschichteten Formkörpers ≥ 50 Gew. % eines Wirkstoffs aus der Gruppe der Ascorbinsäure und ihrer Derivate oder aus der Gruppe der Salicylsäure und deren Derivate, bevorzugt aus der Gruppe der Acetylsalicylsäure und ihrer Derivate enthält.

5. Gefriergetrockneter Formkörper nach einem der Ansprüche 1 bis 4 mit einem Gerüstbildneranteil ≤ 10 Gew. %, bevorzugter ≤ 5 Gew. %, bezogen auf die Gesamtzusammensetzung des gefriergetrockneten beschichteten Formkörpers.

6. Gefriergetrockneter Formkörper nach einem der Ansprüche 1 bis 5 worin keine Gerüstbildner enthalten sind.

7. Gefriergetrockneter Formkörper nach einem der Ansprüche 1 bis 5, worin der Gerüstbildner ausgewählt ist aus der Gruppe der Hydrokolloide, bevorzugt aus der Gruppe der Polysaccharide, Polyaminosaccharide, Glucosaminoglycane und/oder synthetischen Polymere oder Mischungen davon.

8. Gefriergetrockneter Formkörper nach Anspruch 7, worin der Gerüstbildner ein Alginat, bevorzugt ein Natrium-Alginat, Carboxymethylcellulose oder Chitosan oder eine Mischung davon ist.

9. Gefriergetrockneter Formkörper nach einem der Ansprüche 1 bis 8, worin der Anteil der Beschichtung mit einem Filmbildner ≤ 10 Gew.-%, bevorzugt ≤ 7 Gew.-%, bevorzugter ≤ 5 Gew.-%, jeweils bezogen auf die Gesamtzusammensetzung des gefriergetrockneten beschichteten Formkörpers, beträgt.

10. Gefriergetrockneter Formkörper nach einem der Ansprüche 1 bis 9 worin der Filmbildner für die Beschichtung ausgewählt ist aus der Gruppe der hydrophilen Polymere bevorzugt aus der Gruppe der synthetischen Polymere, bevorzugt aus der Gruppe der Vinylpyrrolidon-Vinylacetat-Copolymere.

11. Gefriergetrockneter Formkörper nach einem der Ansprüche 1 bis 10 der bei Flüssigkeitszufuhr in ≤ 30 Sekunden, bevorzugt ≤ 20 Sekunden, bevorzugter ≤ 10 Sekunden, noch bevorzugter ≤ 5 Sekunden vollständig zerfällt.

12. Verfahren zur Herstellung eines gefriergetrockneten beschichteten Formkörpers das **dadurch gekennzeichnet ist, daß** die Beschichtung auf dem gefrorenen Formkörper erfolgt und der beschichtete gefrorene Formkörper anschließend gefriergetrocknet wird.

13. Verfahren zur Herstellung eines gefriergetrockneten Formkörpers das die Schritte umfasst

(a) Harstellon einer wässrigen Lösung oder Suspension eines oder mehrerer Wirkstoffe, gegebenenfalls eines oder mehrerer Gerüstbildner sowie gegebenenfalls eines oder mehrerer Hilfsstoffe,
(b) Giessen der Mischung in eine Form
(c) Gefrieren der Mischung in der Form unter Erhalt von gefrorenen Formkörpern
(d) Entnehmen der gefrorenen Formkörper aus der Form und gegebenenfalls Rondieren
(e) Besprühen der gefrorenen Formkörper mit einer Beschichtungszusammensetzung, enthaltend mindestens einen Filmbildner sowie mindestens ein Lösungs- und/oder Dispergiermittel
(f) Gefriertrocknung der mit dem Filmbildner beschichteten gefrorenen Formkörper unter Bildung des gefriergetrockneten Formkörpers.

14. Verwendung des gefriergetrockneten Formkörpers nach einem der Ansprüche 1 bis 11 als kosmetisches Mittel oder als pharmazeutisches Mittel.

15. Kit-of-parts Kombination enthaltend mindestens einen gefriergetrockneten Formkörper gemäß einem der Ansprüche 1 bis 11 sowie mindestens eine wässrige Lösung, die einen oder mehrere Wirkstoffe und/oder gegebenenfalls einen oder mehrere Hilfsstoffe enthält, in zusammengehöriger, räumlicher Anordnung.

Europäisches Patentamt
European Patent Office
Office européen des brevets

# EUROPÄISCHER RECHERCHENBERICHT

Nummer der Anmeldung
EP 10 16 0418

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2004/035023 A (SUWELACK SKIN & HEALTH CARE AG [DE]; FRAHLING STEFAN [DE]; MALESSA RAL) 29. April 2004 (2004-04-29)<br>* Seite 3, Zeile 19 - Seite 4, Zeile 6 *<br>* Seite 5, Zeilen 8-15 *<br>* Seite 6, Zeilen 27-31 *<br>* Seite 7, Zeile 31 *<br>* Seite 10, Zeile 10 *<br>* Seite 14, Zeilen 8-26; Beispiele *<br>----- | 1-15 | INV.<br>A61K9/16<br>A61K9/19<br>A61K9/20<br>A61K9/28 |
| X | EP 0 701 815 A (ALFATEC PHARMA GMBH [DE]) 20. März 1996 (1996-03-20)<br>* Seite 3, Zeilen 11-28 *<br>* Seite 6, Zeile 52 - Seite 7, Zeile 5 *<br>* Beispiele *<br>----- | 1-15 | |
| X | GB 1 206 033 A (GRACE W R & CO [US]) 23. September 1970 (1970-09-23)<br>* das ganze Dokument *<br>* Abbildung 4 *<br>----- | 1,6 | |
| | | | RECHERCHIERTE SACHGEBIETE (IPC) |
| Y,D | EP 0 081 912 A (WYETH JOHN & BROTHER LTD [GB]) 22. Juni 1983 (1983-06-22)<br>* Seite 4, Zeilen 29-35 *<br>* Beispiele *<br>----- | 1-15 | A61K |
| Y,D | DATABASE WPI Week 200443<br>Thomson Scientific, London, GB; AN 2004-454129<br>XP002544732<br>& JP 2004 149468 A (NIKKO SEIYAKU KK) 27. Mai 2004 (2004-05-27)<br>* Zusammenfassung *<br>----- | 1-15 | |

Der vorliegende Recherchenbericht wurde für alle Patentansprüche erstellt

1

| Recherchenort | Abschlußdatum der Recherche | Prüfer |
|---|---|---|
| München | 2. September 2010 | Villa Riva, A |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

**ANHANG ZUM EUROPÄISCHEN RECHERCHENBERICHT ÜBER DIE EUROPÄISCHE PATENTANMELDUNG NR.** EP 10 16 0418

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

02-09-2010

| Im Recherchenbericht angeführtes Patentdokument | Datum der Veröffentlichung | Mitglied(er) der Patentfamilie | Datum der Veröffentlichung |
|---|---|---|---|
| WO 2004035023 A | 29-04-2004 | AU 2003276011 A1 | 04-05-2004 |
| | | EP 1551373 A1 | 13-07-2005 |
| | | JP 2006504799 T | 09-02-2006 |
| | | TW 262796 B | 01-10-2006 |
| | | US 2005281849 A1 | 22-12-2005 |
| EP 0701815 A | 20-03-1996 | GR 3030675 T3 | 30-11-1999 |
| GB 1206033 A | 23-09-1970 | BE 709590 A | 30-05-1968 |
| | | CH 483609 A | 31-12-1969 |
| | | DE 1729206 A1 | 27-05-1971 |
| | | FR 1555917 A | 31-01-1969 |
| | | IE 31884 B1 | 07-02-1973 |
| | | NL 6801034 A | 25-07-1968 |
| | | SE 340033 B | 01-11-1971 |
| EP 0081912 A | 22-06-1983 | AR 229543 A1 | 15-09-1983 |
| | | AU 554816 B2 | 04-09-1986 |
| | | AU 9061882 A | 09-06-1983 |
| | | BR 8206982 A | 11-10-1983 |
| | | CA 1200960 A1 | 25-02-1986 |
| | | CS 238637 B2 | 16-12-1985 |
| | | CY 1326 A | 27-06-1986 |
| | | DD 206534 A1 | 01-02-1984 |
| | | DE 3276260 D1 | 11-06-1987 |
| | | DK 532682 A | 03-06-1983 |
| | | DZ 481 A1 | 13-09-2004 |
| | | EG 15502 A | 30-12-1986 |
| | | ES 8401720 A1 | 16-03-1984 |
| | | FI 823983 A | 03-06-1983 |
| | | GB 2119246 A | 16-11-1983 |
| | | GR 77773 A1 | 25-09-1984 |
| | | HK 19586 A | 27-03-1986 |
| | | HU 187802 B | 28-02-1986 |
| | | IE 53696 B1 | 18-01-1989 |
| | | IL 67266 A | 30-06-1985 |
| | | IN 157896 A1 | 19-07-1986 |
| | | JP 1049242 B | 24-10-1989 |
| | | JP 1568399 C | 10-07-1990 |
| | | JP 58113123 A | 05-07-1983 |
| | | KE 3601 D | 14-02-1986 |
| | | MA 19658 A1 | 01-07-1983 |
| | | MX 195411 A | 01-11-1993 |
| | | MY 50286 A | 31-12-1986 |
| | | NO 824023 A | 03-06-1983 |
| | | NZ 202482 A | 30-08-1985 |

EPO FORM P0461

Für nähere Einzelheiten zu diesem Anhang : siehe Amtsblatt des Europäischen Patentamts, Nr.12/82

**ANHANG ZUM EUROPÄISCHEN RECHERCHENBERICHT ÜBER DIE EUROPÄISCHE PATENTANMELDUNG NR.** EP 10 16 0418

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

02-09-2010

| Im Recherchenbericht angeführtes Patentdokument | Datum der Veröffentlichung | Mitglied(er) der Patentfamilie | Datum der Veröffentlichung |
|---|---|---|---|
| EP 0081912 A | | PH 18441 A | 08-07-1985 |
| | | PL 239301 A1 | 15-08-1983 |
| | | PT 75923 A | 01-12-1982 |
| | | SU 1514239 A3 | 07-10-1989 |
| | | US 4758598 A | 19-07-1988 |
| | | YU 265582 A1 | 20-03-1985 |
| | | ZA 8208371 A | 27-06-1984 |
| | | ZW 25282 A1 | 20-06-1984 |
| JP 2004149468 A | 27-05-2004 | JP 4377123 B2 | 02-12-2009 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- JP 2004149468 A
- EP 0081912 A
- US 4305502 A
- DE 69227467
- JP 2003238693 A
- WO 04011537 A
- WO 05073296 A
- US 5843347 A
- US 5578307 A
- US 5405616 A
- EP 0701815 A
- DE 4201179
- GB 1206033 A
- JP 54105289 B
- DE 10248314
- WO 2004035023 A
- DE 4328329 C2
- DE 4028622 C2
- DE 10350654 A1


### In der Beschreibung aufgeführte Nicht-Patentliteratur


- Aqueous Film Coating. **Seitz, J.A. ; J.Swarbrick ; J.C. Boylan.** Encyclopedia of pharmaceutical technology. Marcel Dekker, 1988, vol. 1, 337-349
- Introduction and overview of pharmaceutical coating. **Cole, G.C. ; G. Cole ; J. Hogan ; M. Aulton.** Pharmaceutical Coating Technology. Taylor and Francis LTD, 1995, 1-5
- **Aulton M. ; G. Cole.** Mechanical Properties of film coats, Pharmaceutical coating technology. Taylor and Francis, 1995, 280-362